# DEMANDE DE BREVET EUROPEEN

(11) **EP 2 236 495 A1**
(43) Date de publication de la demande: **06.10.2010**
(21) Numéro de dépôt: 10075301.1
(22) Date de dépôt: 19.09.2008
(51) Int. Cl.: C07D 207/09, C07D 209/42, C07D 209/52, C07D 217/26, C07D 233/36, C07D 271/08, A61K 31/385, A61K 31/40, A61K 31/403, A61K 31/404, A61K 31/4245, A61K 31/472, C07D 495/10

(54) **Nouveaux sels d'addition d'inhibiteurs d'enzyme de conversion de l'angiotensine à des acides donneurs de NO, leur procédé de préparation et les compositions pharmaceutiques qui les contiennent**

(30) Priorité: 21.09.2007 FR 0706629
(62) Demande divisionnaire de: 08290885.6
(71) Demandeur: Les Laboratoires Servier, 92284 Suresnes Cedex (FR)
(72) Inventeur: De Nanteuil, Guillaume, 92150 Suresnes (FR); Portevin, Bernard, 45450 Fay aux Loges (FR); Gloanec, Philippe, 78160 Marly le Roi (FR); Parmentier, Jean-Gilles, 92130 Issy les Moulineaux (FR); Benoist, Alain, 95130 Franconville (FR); Verbeuren, Ton, 78540 Vernouillet (FR); Rupin, Alain, 37510 Savonnières (FR); Courchay, Christine, 91430 Igny (FR); Simonet, Serge, 78700 Conflans Sainte Honorine (FR)
(74) Mandataire: Giudicelli, Cathy

(57) **Abrégé**

Composés de formule (I) :

(A)ₘ • (B)ₙ (I)

dans laquelle A représente un composé inhibiteur de l'enzyme de conversion de l'angiotensine comportant au moins une fonction basique salifiable, B représente un composé comportant au moins une fonction acide salifiable et au moins un groupement donneur de NO, m représente le nombre de fonctions acides salifiées de B et n représente le nombre de fonctions basiques salifiées de A,
étant entendu que la ou les liaisons entre A et B sont de type ionique.
Médicaments.

## Description

La présente invention concerne de nouveaux sels d'addition d'inhibiteurs d'enzyme de conversion de l'angiotensine à des acides donneurs de NO, leur procédé de préparation et les compositions pharmaceutiques qui les contiennent.

Ces composés trouvent leur application dans le domaine de l'hypertension et des maladies cardiovasculaires.

L'hypertension entraîne un risque élevé d'accidents vasculaires, en particulier au niveau cérébral et coronaire. Elle est de plus en plus fréquemment associée à d'autres pathologies comme l'athérosclérose ou des maladies métaboliques telles que l'obésité, le diabète ou l'insuffisance rénale, ce qui augmente considérablement le risque de spasmes et de thromboses.

Depuis la découverte en 1980 de son action cardiovasculaire, le monooxyde d'azote (NO) est reconnu comme une molécule vasodilatatrice et vasoprotectrice capable de prévenir les vasospasmes, l'athérosclérose et la thrombose, ce médiateur offrant ainsi une protection contre les maladies cardiovasculaires. Le NO est essentiellement produit par l'endothélium. En général, le NO est reconnu d'avoir des propriétés vasodilatatrices, anti-adhésives, anti-thrombotiques, anti-inflammatoires et anti-oxydantes.

Les inhibiteurs de l'enzyme de conversion de l'angiotensine (IEC), comme le perindopril (Ferrari et al., 2005, Am J Hypertension, 18, 142S-154S), sont des agents thérapeutiques dont les effets protecteurs cardiovasculaires sont bien reconnus ; ces produits diminuent la pression artérielle, l'infarctus du myocarde, l'insuffisance cardiaque, la dysfonction ventriculaire gauche, le stroke et la mortalité cardiovasculaire. Ces effets bénéfiques sont également observés chez les patients diabétiques, avec ou sans athérosclérose. Pour le perindopril, les données cliniques récentes ont mis en évidence ses propriétés anti-athéromateuses et anti-inflammatoires et ses effets bénéfiques sur le dysfonctionnement endothélial (Ferrari et al., 2005). En bloquant l'enzyme de conversion, les IEC (1) préviennent la formation de l'angiotensine II, un puissant vasoconstricteur qui est impliqué dans les maladies cardiovasculaires (Kon et Jabs, 2004, Current Opinion Nephrol Hypertens, 13, 291-297 ; Unger, 2002, Am J Cardiol, 89, 3A-10A ; Ferrari et al., 2005) et (2) protègent la bradykinine de sa dégradation, la bradykinine dont les effets bénéfiques cardiovasculaires (par ex effets anti-ischémiques) sont dus à la production endothéliale de NO (Unger 2002 ; Ferrari et al., 2005).

Il est bien établi que dans plusieurs conditions pathologiques telles que l'athérosclérose, l'hypertension, le diabète et d'autres, la production de NO est diminuée voir même complètement absente (Gewaltig and Kodja, 2002 , Cardiovasc Res, 55, 250-260 ; Russo et al, 2002, Vasc Pharmacol, 38, 259-269). Dans cette condition de dysfonctionnement endothélial, les effets bénéfiques des IEC liés à l'inhibition de la dégradation de la bradykinine seront moins exprimés. Il a été postulé que des produits hybrides, inhibiteurs IEC et donneurs de NO pourraient être bénéfiques dans ces différentes conditions pathologiques, plus spécifiquement dans les pathologies cardiovasculaires.

De plus, des composés donneurs de NO, tels que la nitroglycérine, sont utilisés depuis longtemps pour soigner l'angine de poitrine et l'insuffisance cardiaque. L'effet bénéfique de ces produits est lié à leur capacité de former (de façon spontanée ou de façon métabolique) du NO. L'utilisation de ces produits a aussi conduit à l'observation que chez le sujet hypertendu, ces donneurs de NO provoquent une baisse prédominante de la pression artérielle systolique (Nesbitt, 2005, Hypertension, 45, 352-353). Une pression artérielle systolique non controlée est un facteur de risque important pour les accidents cérébraux et cardiaques et elle est souvent résistante aux traitements des anti-hypertenseurs. En effet, malgré des effets démontrés antihypertenseurs et vasoprotecteurs des produits tels que les IEC et d'autres classes de produits anti-hypertenseurs, la pression artérielle, en particulier systolique, reste difficile à contrôler et le taux de morbidité et mortalité reste élevé chez les sujets hypertendus.

Ajouter donc à des IEC tels que le perindopril, des propriétés donneurs de NO apparaît comme une stratégie importante dans la lutte contre les maladies cardiovasculaires. En effet, avoir des propriétés donneurs de NO améliorerait les propriétés antihypertensives, cardio-et vasculoprotectrices, le NO étant une molécule vasodilatatrice, anti-plaquettaire (donc anti-thrombotique), anti-adhésive et anti-oxydante (Waldorf et al. 2003, J Thromb Haemost, 1, 2112-2118 ; Gewaltig and Kodja, 2002, Cardiovasc Res, 55, 250-260).

Les composés de la présente invention présentent cette double activité pharmacologique leur conférant des propriétés intéressantes dans le domaine de l'hypertension et des pathologies cardiovasculaires.

Plus spécifiquement, la présente invention concerne les sels de formule (I) :

(A)ₘ • (B)n (I)

dans laquelle A représente un composé inhibiteur de l'enzyme de conversion de l'angiotensine comportant au moins une fonction basique salifiable, B représente un composé comportant au moins une fonction acide salifiable et au moins un groupement donneur de NO, m représente le nombre de fonctions acides salifiées de B et n représente le nombre de fonctions basiques salifiées de A,
étant entendu que la ou les liaisons entre A et B sont de type ionique.

Par fonction basique salifiable, on entend une fonction susceptible d'accepter un proton.

A titre d'exemple de fonction basique salifiable, on citera une amine primaire, secondaire ou tertiaire.

Par fonction acide salifiable, on entend une fonction susceptible de libérer un proton.

A titre d'exemple de fonction acide salifiable, on citera un groupement -CO₂H, -SO₃H ou P(O)(OH)₂.

Par groupement donneur de NO, on entend un groupement susceptible de donner, libérer et/ou transférer du monoxyde d'azote ou de l'une de ses formes biologiquement actives, et/ou de stimuler in vivo la production endogène de monoxyde d'azote ou de l'une de ses formes biologiquement actives.

De façon préférentielle, l'inhibiteur A de l'enzyme de conversion de l'angiotensine appartient à la formule (II) suivante : 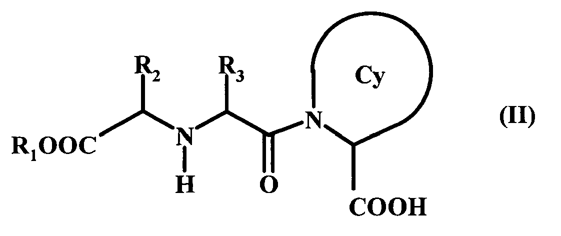 dans laquelle :
- R₁ représente un atome d'hydrogène ou un groupement alkyle C₁-C₆ linéaire ou ramifié,
- R₂ représente un groupement alkyle C₁-C₆ linéaire ou ramifié ou aryl-(C₁-C₆) alkyle dans lequel le groupement alkyle est linéaire ou ramifié,
- R₃ représente un groupement alkyle C₁-C₆ linéaire ou ramifié ou amino-(C₁-C₆) alkyle dans lequel le groupement alkyle est linéaire ou ramifié, 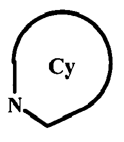 représente un système mono- ou bicyclique azoté comportant de 3 à 12 atomes de carbone et pouvant comporter un ou plusieurs hétéroatomes supplémentaires choisis parmi N, O et S, ledit système étant éventuellement substitué par un ou plusieurs groupement choisis parmi alkyle C₁-C₆ linéaire ou ramifié, alkoxy C₁-C₆ linéaire ou ramifié, oxo, carboxy, aryle, cycloalkyle C₃-C₈, hétéroaryle, carboxy-(C₁-C₆) alkyle dans lequel le groupement alkyle est linéaire ou ramifié, hydroxy,
   étant entendu que lorsque le système azoté est bicyclique, il peut être fusionné, spiranique ou ponté. 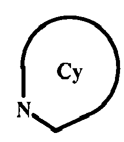
représente préférentiellement un cycle pyrrolidine, perhydroindole, octahydro-cyclopenta[b]pyrrole, imidazolidine ou tétrahydroisoquinoléine.

Parmi les inhibiteurs A de l'enzyme de conversion de l'angiotensine, on peut citer à titre d'exemple l'énalapril de formule (IIa), l'énalaprilate de formule (IIb), le lisinopril de formule (IIc), le perindopril de formule (IId), le perindoprilate de formule (IIe), le ramipril de formule (IIf), le spirapril de formule (IIg), le trandolapril de formule (IIh), le trandolaprilate de formule (IIi), l'imidapril de formule (IIj), le moexipril de formule (IIk), le quinapril de formule (IIm) ou le ramiprilate de formule (IIn).

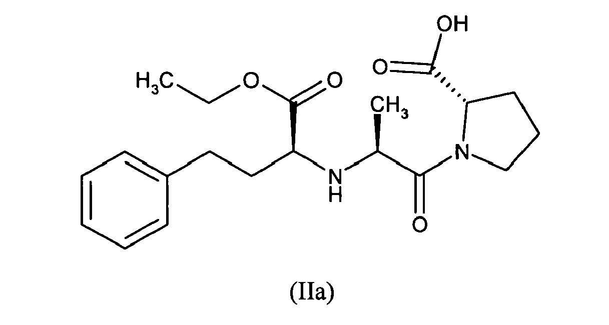

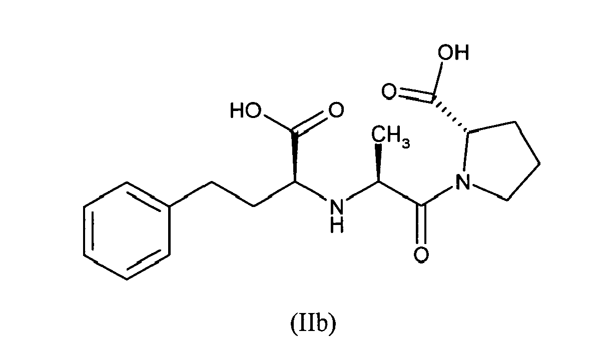

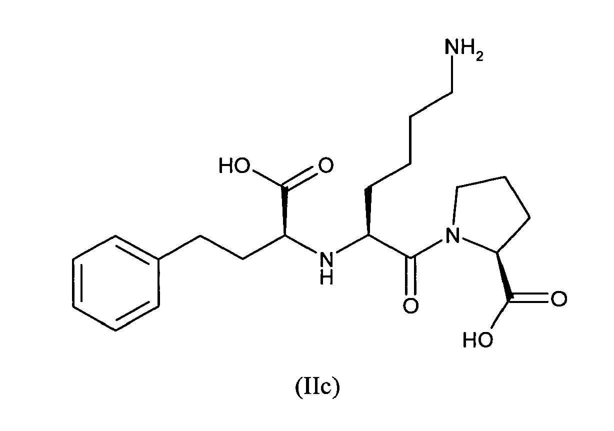

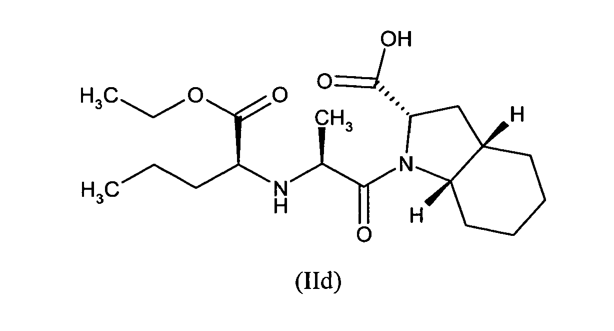

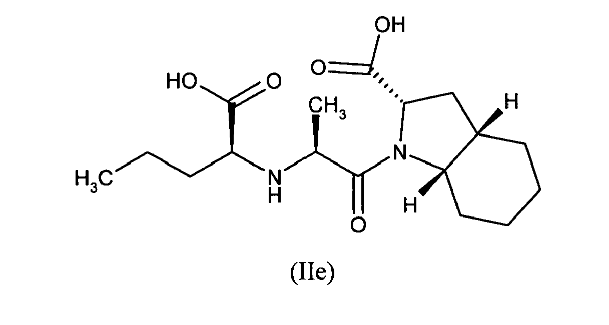

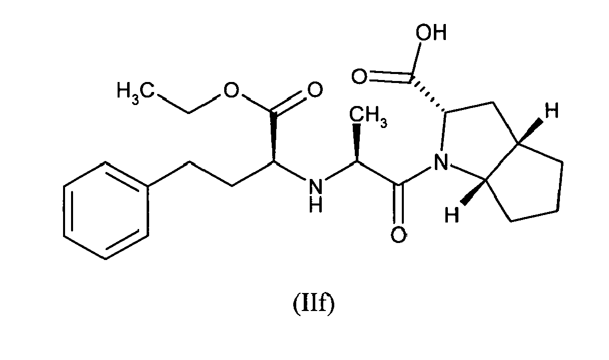

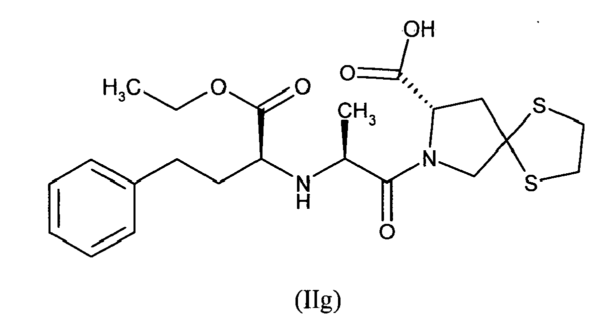

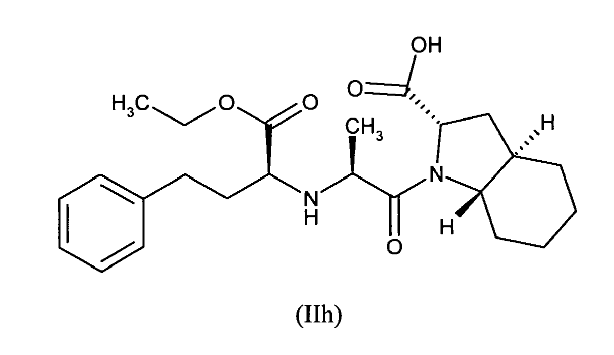

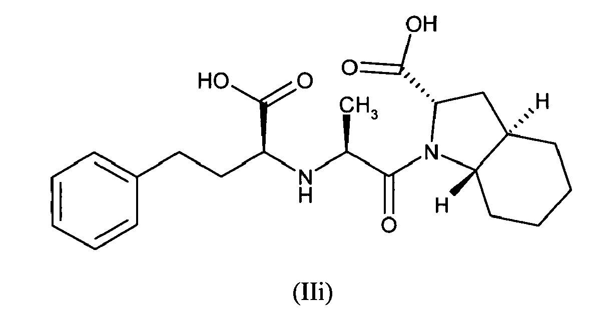

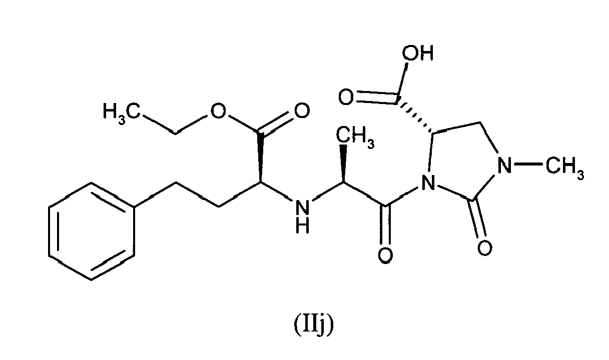

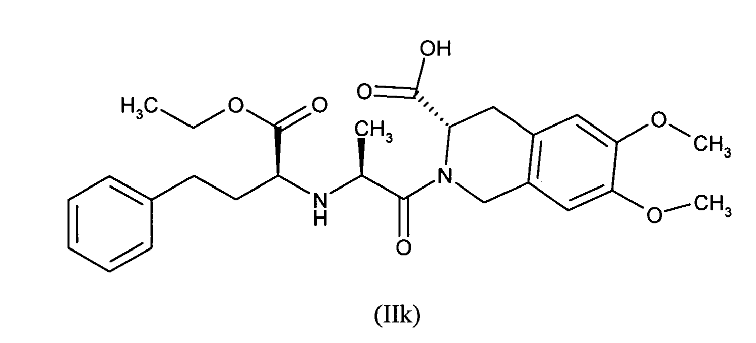

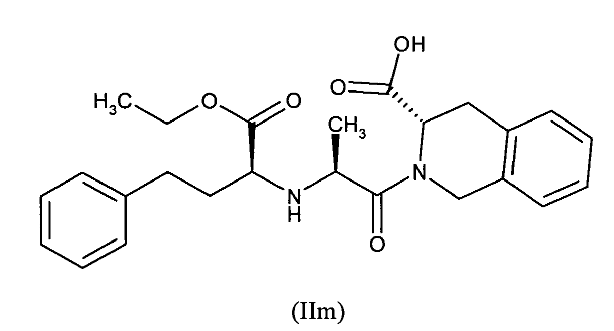

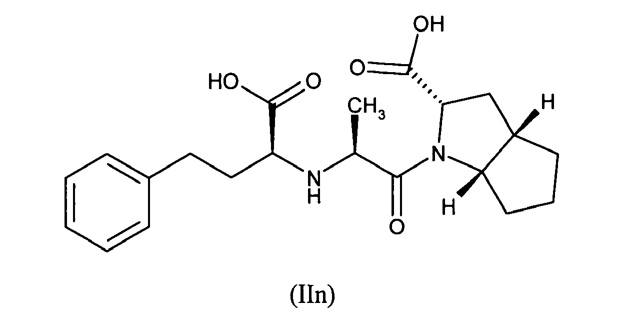

Les composés préférés de formule (I) sont les composés suivants :

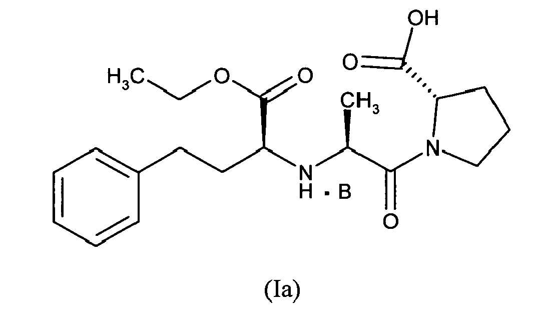

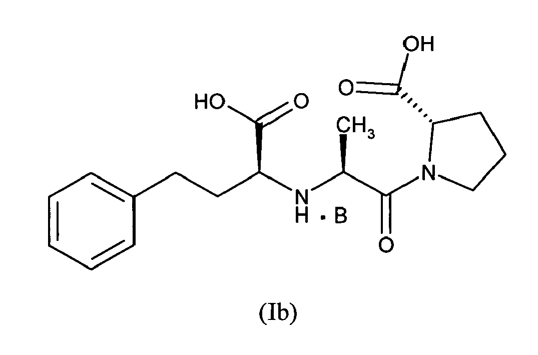

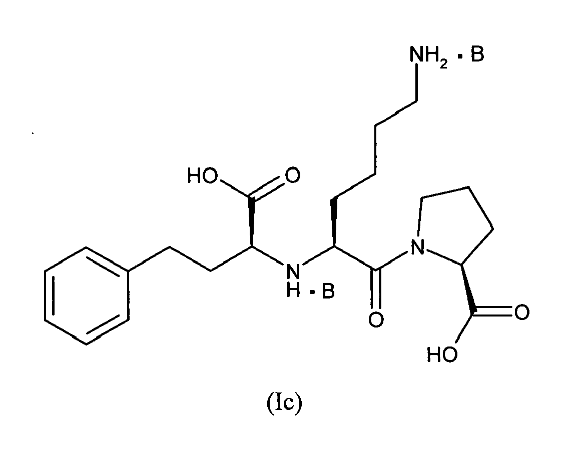

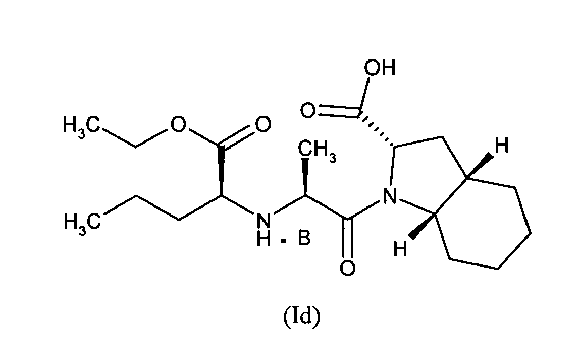

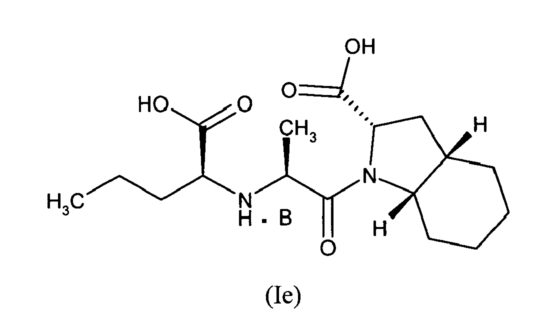

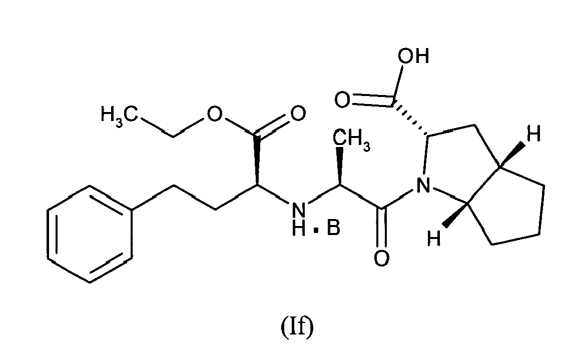

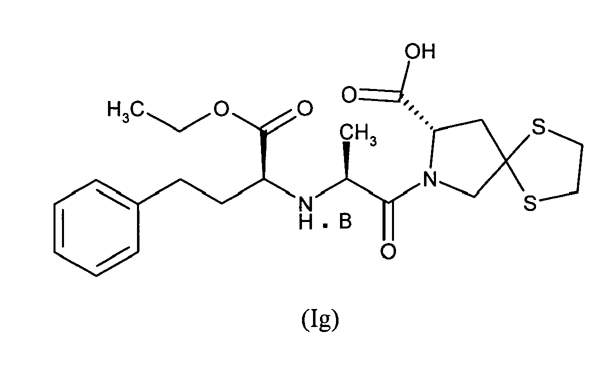

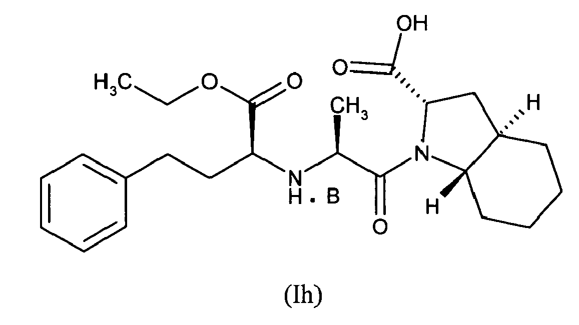

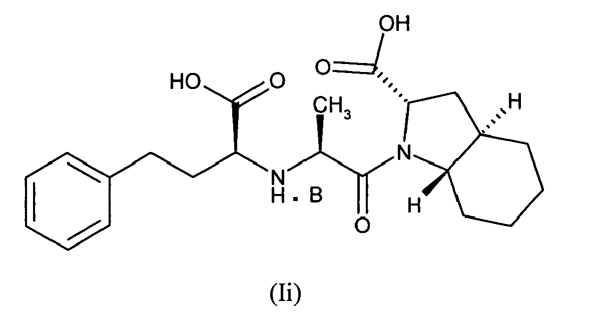

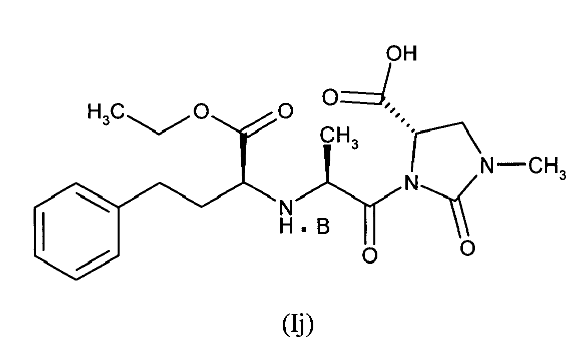

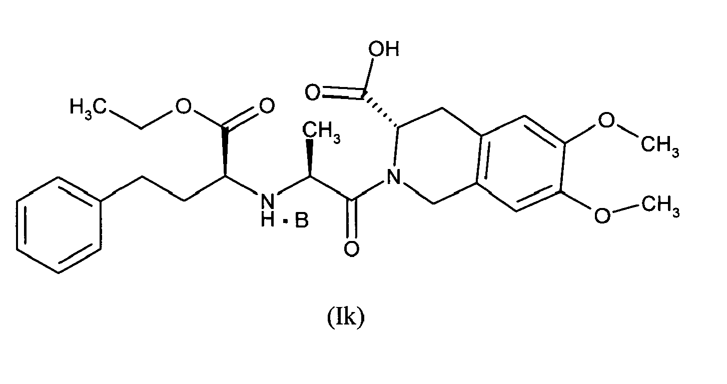

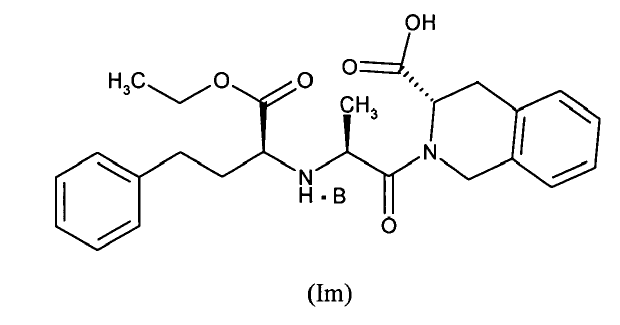

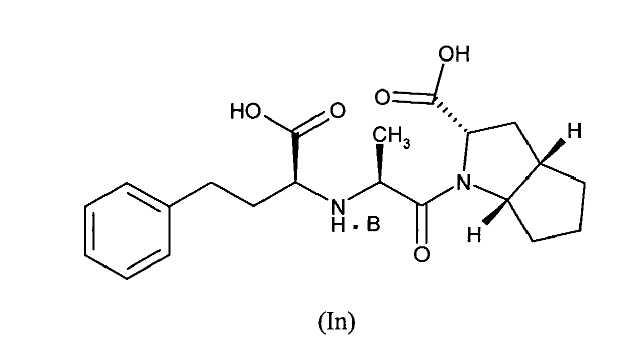

De façon préférentielle, le composé B appartient à la formule (III) suivante :

X-(Ak₁)ₓ-(Y)_{y}-(Ak₂)_{z}-Z (III)

dans laquelle :
- X représente un groupement CO₂H, SO₃H ou P(O)(OH)₂,
- Ak₁ et Ak₂, identiques ou différents, représentent chacun un groupement alkylène C₁-C₈ linéaire ou ramifié, saturé ou insaturé, dans lequel un ou plusieurs des atomes de carbone peuvent être remplacés par un atome d'oxygène, de soufre, d'azote ou un groupement SO₂, ledit groupement alkylène étant éventuellement substitué par un ou plusieurs groupements choisis parmi CO₂H, SO₃H, hydroxy et amino,
- x, y et z, identiques ou différents, représentent chacun 0 ou 1,
- Y représente CO ou CONH,
- Z représente un groupement donneur de NO.

Parmi les composés de formule (III), on peut citer les composés appartenant à l'une des formules suivantes :

HO₂C-(Ak₁)ₓ-Z (IIIa)

HO₂C-CH=CH-(Ak₃)ₓ-Z (IIIb)

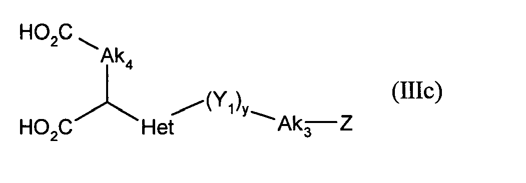

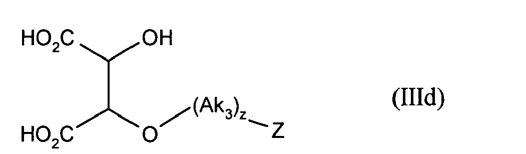

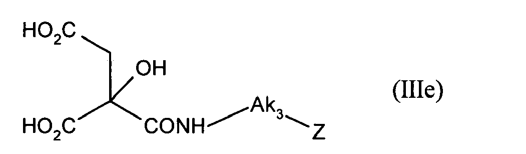

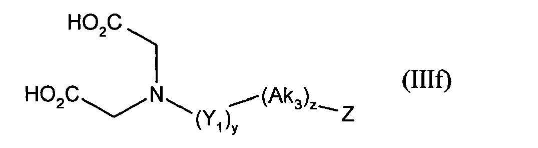

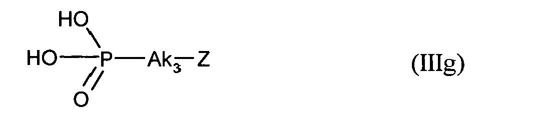

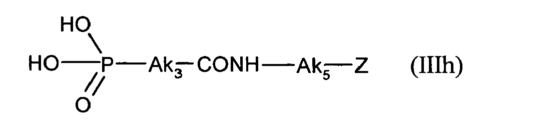

HO₃S-Ak₆-CONH-Ak₅-Z (IIIi)

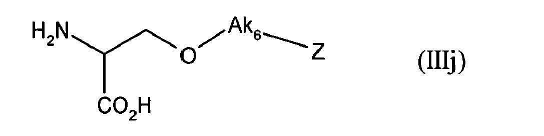

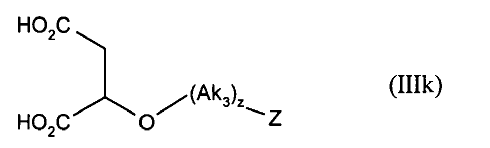

dans lesquelles :
Ak₁, x, y, z et Z sont tels que définis précédemment,
Ak₃ et Ak₅, identiques ou différents, représentent chacun un groupement alkylène C₁-C₆ linéaire ou ramifié saturé dans lequel un ou plusieurs des atomes de carbone peuvent être remplacés par un atome d'oxygène, de soufre, d'azote ou un groupement SO₂,
Ak₄ représente un groupement alkylène C₁-C₃ linéaire ou ramifié saturé dans lequel un des atomes de carbone peut être remplacé par un atome d'oxygène, de soufre, d'azote ou un groupement SO₂,
Ak₆ représente un groupement alkylène C₂-C₅ linéaire ou ramifié saturé dans lequel un des atomes de carbone peut être remplacé par un atome d'oxygène, de soufre, d'azote ou un groupement SO₂,
Het représente O ou NH,
et Y₁ représente un groupement carbonyle.

De façon préférentielle, Z est choisi parmi les groupements suivants : 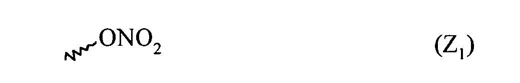 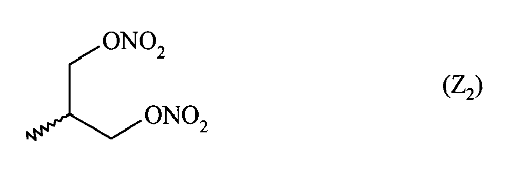 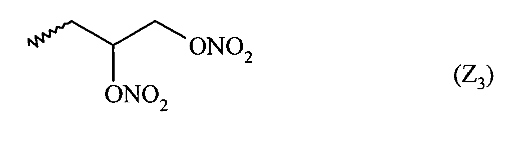 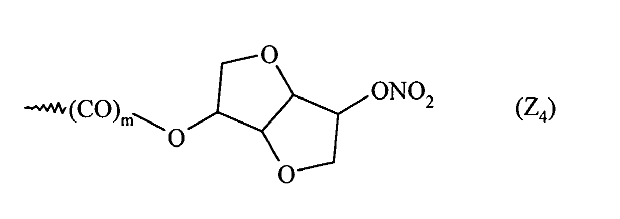 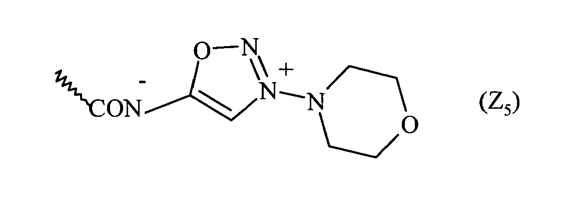 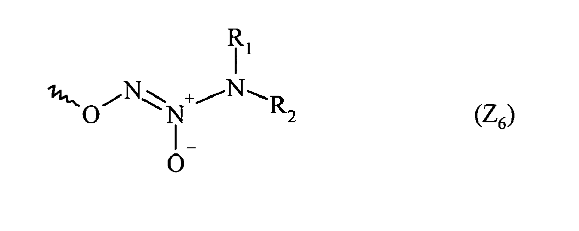 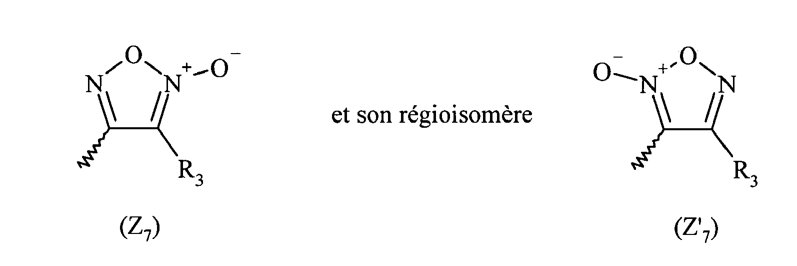 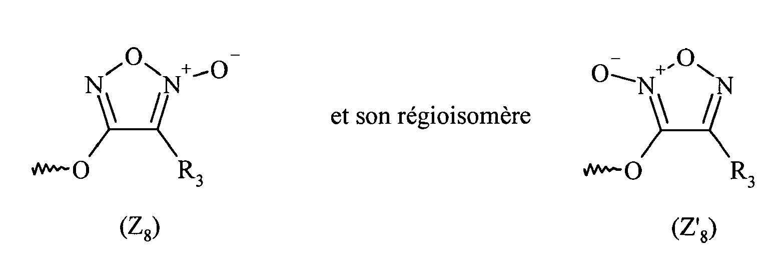 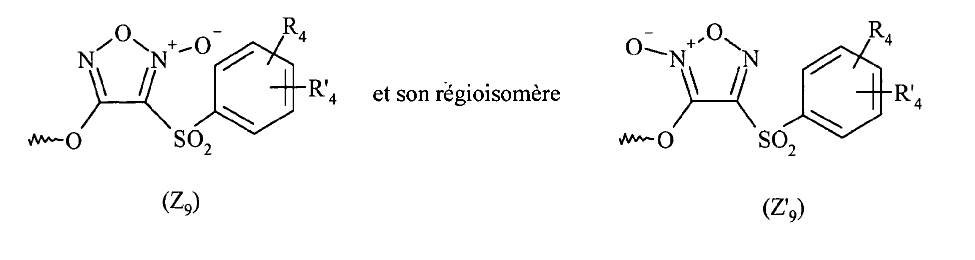 dans lesquels :
m représente 0 ou 1,
R₁ et R₂ , identiques ou différents, représentent chacun un groupement alkyle C₁-C₆ linéaire ou ramifié, ou bien R₁ et R₂ forment ensemble, avec l'atome d'azote qui les portent, un hétérocycle azoté comportant de 3 à 7 chaînons, éventuellement substitué par un groupement alkyle C₁-C₆ linéaire ou ramifié,
R₃ représente un groupement méthyle ou aminocarbonyle,
et R₄ et R'₄, identiques ou différents, représentent chacun un atome d'hydrogène ou d'halogène ou un groupement alkyle C₁-C₆ linéaire ou ramifié, trifluorométhyle ou trifluorométhoxy.

Par hétérocycle azoté comportant de 3 à 7 chaînons, on entend un groupement monocyclique saturé de 3 à 7 chaînons contenant un, deux ou trois hétéroatomes, l'un de ces hétéroatomes étant l'atome d'azote, et le ou les hétéroatomes supplémentaires éventuellement présents étant choisis parmi les atomes d'oxygène, d'azote ou de soufre.

L'hétérocycle azoté de 3 à 7 chaînons préféré est le groupement pyrrolidinyle.

Les composés B préférés sont les suivants :

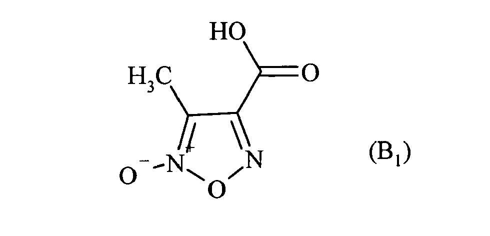

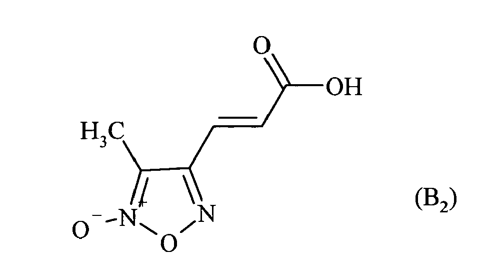

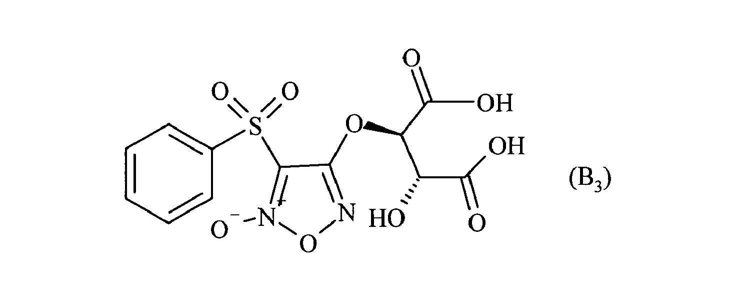

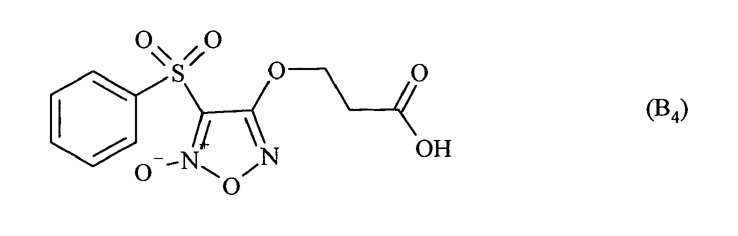

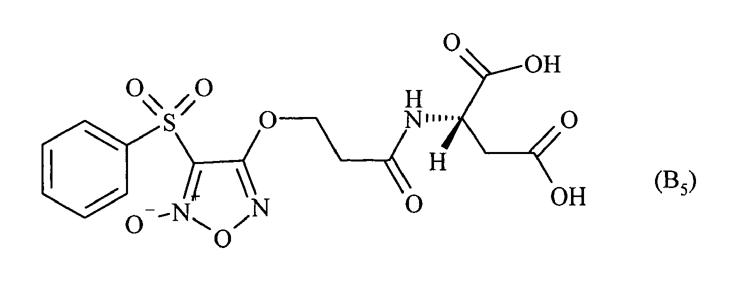

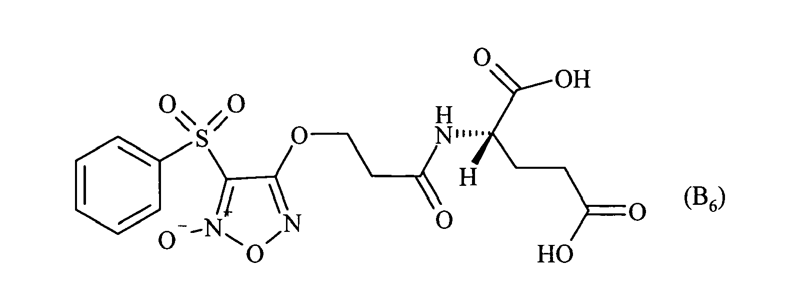

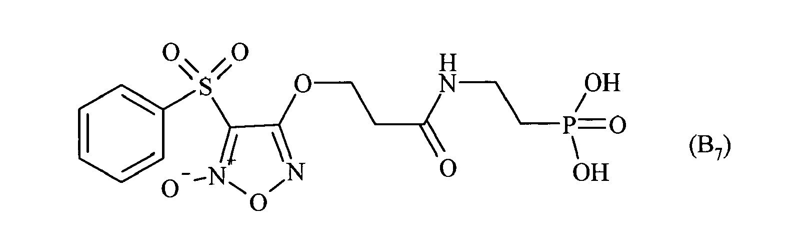

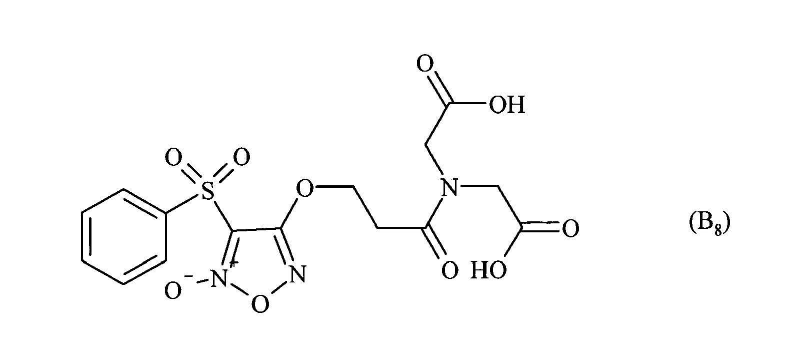

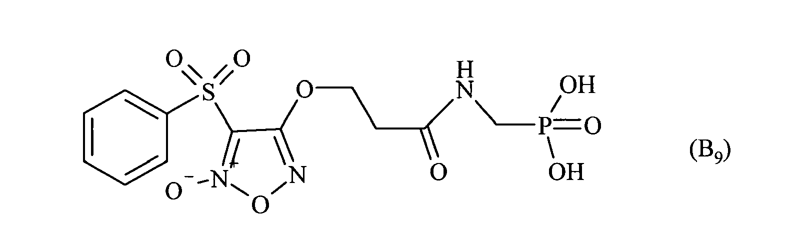

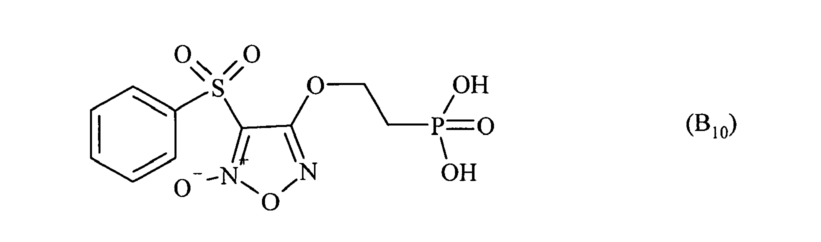

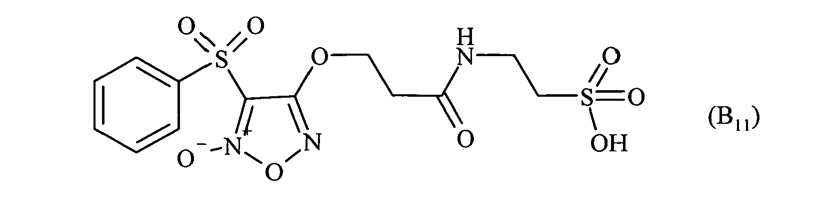

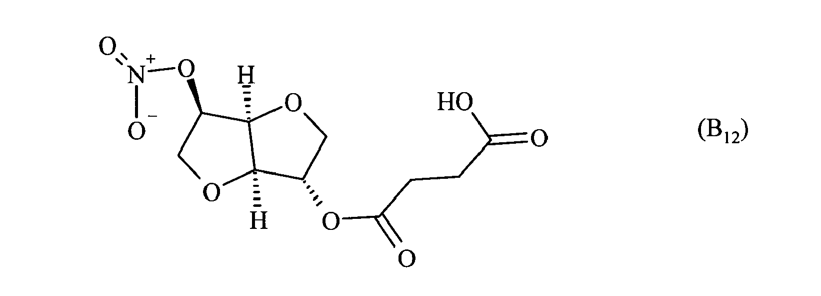

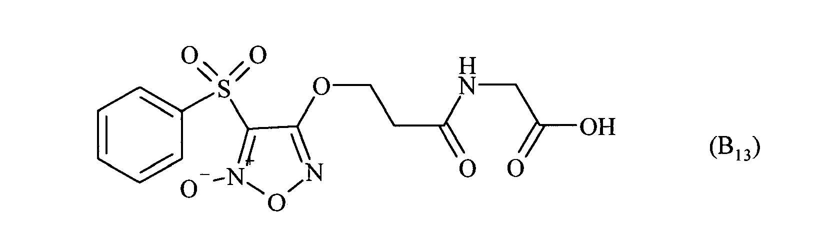

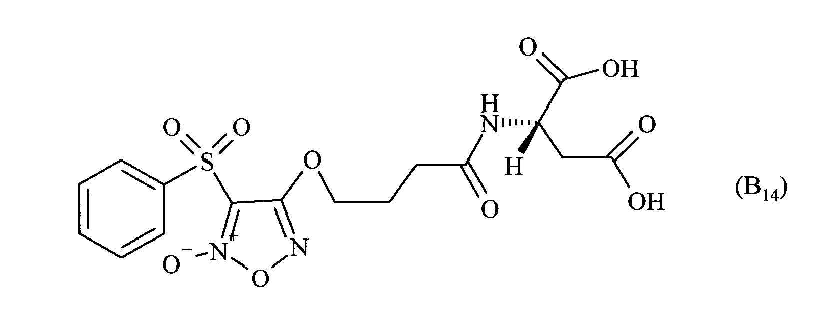

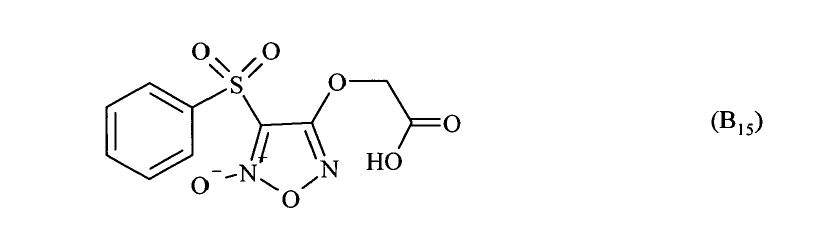

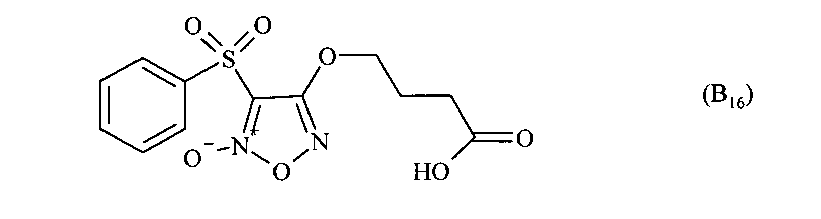

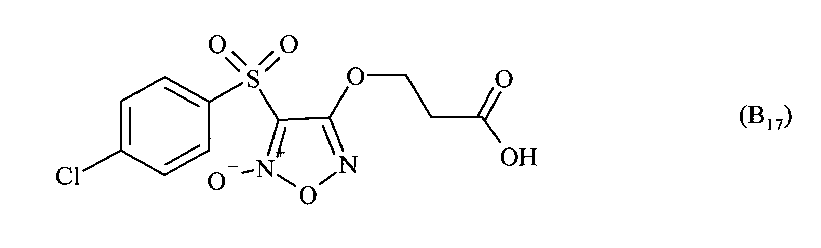

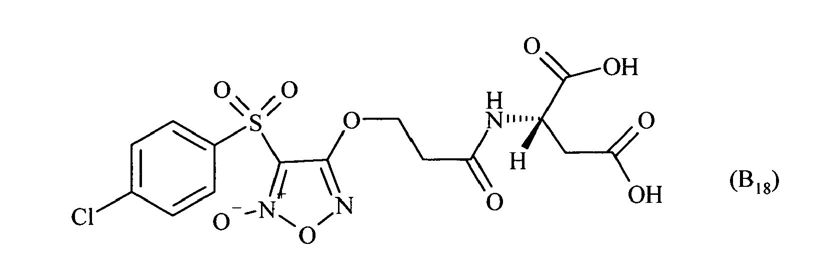

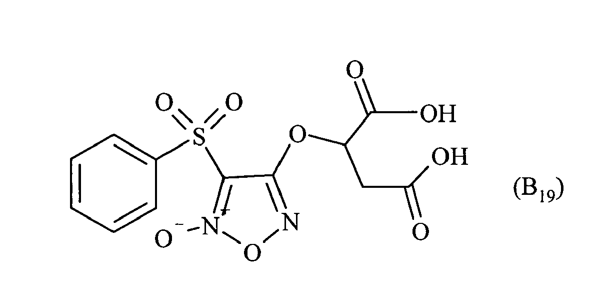

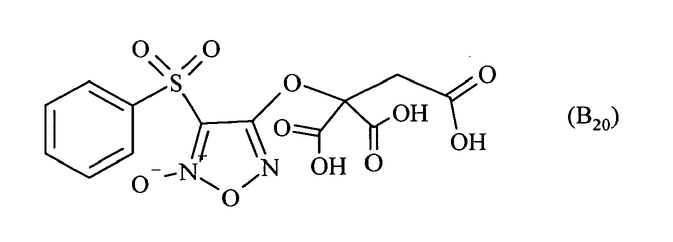

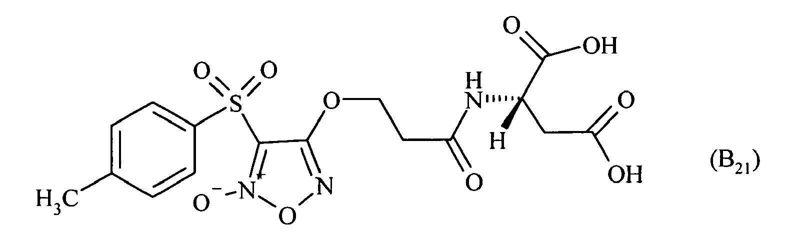

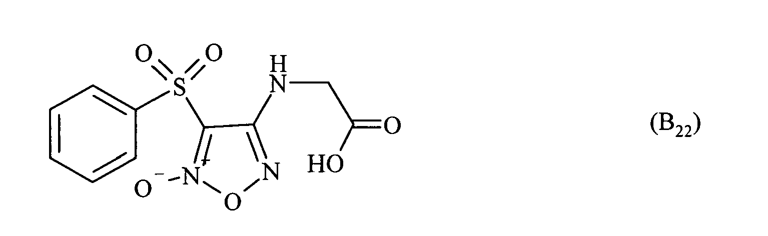

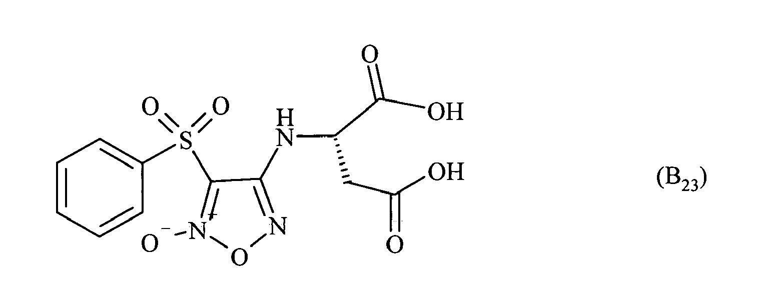

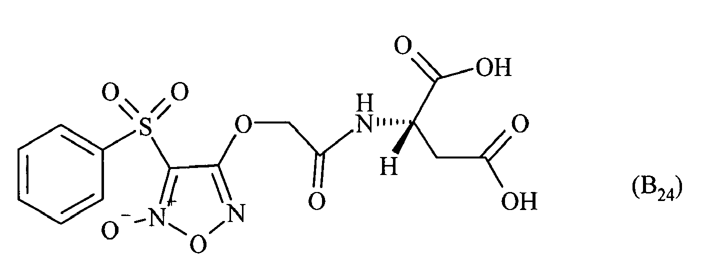

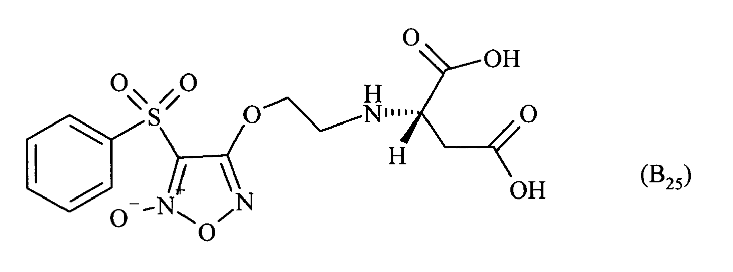

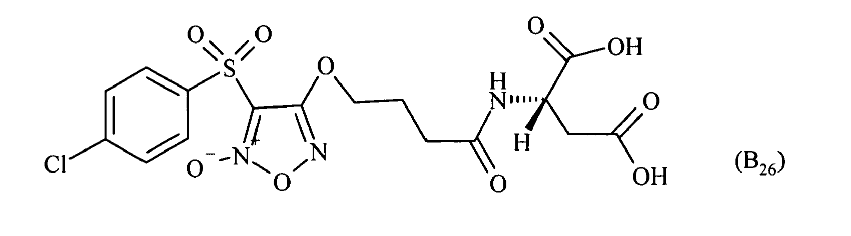

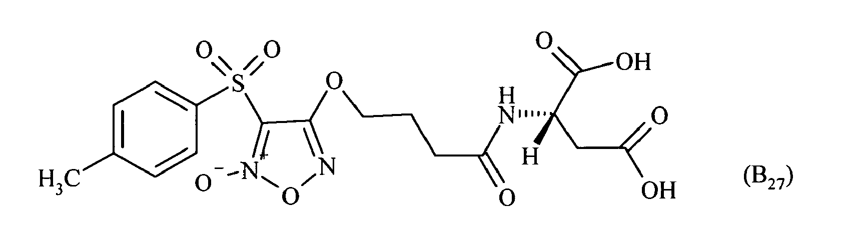

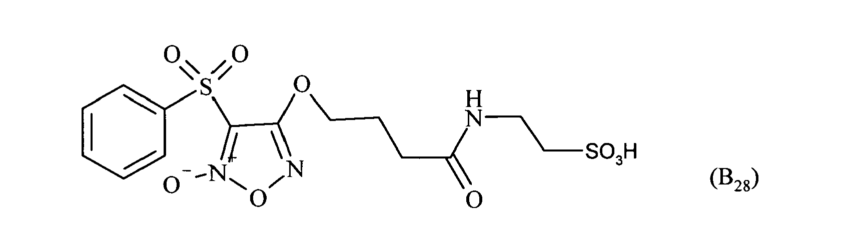

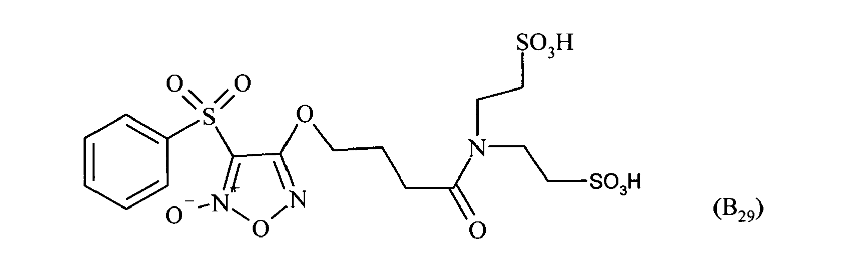

Les composés préférés de formule (I) sont les suivants :
- Acide (2*S*)-2-[(3-{[5-oxydo-4-(phénylsulfonyl)-1,2,5-oxadiazol-3-yl]-oxy}-propionyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1);
- Acide (2*S*)-2-[(3-{[5-oxydo-4-(phénylsulfonyl)-1,2,5-oxadiazol-3-yl]-oxy}-propionyl)-amino]-pentanedioique - Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique (1:1);
- Acide 2-[(3-{[5-oxydo-4-(phénylsulfonyl)-1,2,5-oxadiazol-3-yl]-oxy}-propionyl)-amino]-éthanesulfonique - Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique (1:1);
- Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique - Acide {[(3-{[5-oxydo-4-(phénylsulfonyl)-1,2,5-oxadiazol-3-yl]-oxy}-propionyl)-amino]-méthyl}-phosphonique (1:1);
- Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique - Acide 2,2'-[(3-{[5-oxydo-4-(phénylsulfonyl)-1,2,5-oxadiazol-3-yl]-oxy}-propionyl)-imino]-diacétique (1:1);
- Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique - Acide {2-[(3-{[5-oxydo-4-(phénylsulfonyl)-1,2,5-oxadiazol-3-yl]-oxy}-propionyl)-amino]-éthyl}-phosphonique (1:1);
- Acide (2*S*,3a*S*,7a*S*)-1-[(2*S*)-2-{[(1*S*)-1-(éthoxycarbonyl)-butyl]-amino}-propionyl]-octahydro-1*H*-indole-2-carboxylique - Acide (2-{[5-oxydo-4-(phénylsulfonyl)-1,2,5-oxadiazol-3-yl]-oxy}-éthyl)-phosphonique (1:1).;
- Acide (2S)-2-[(4-{[5-oxydo-4-(phénylsulfonyl)-1,2,5-oxadiazol-3-yl]-oxy}-butanoyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)
- Acide (2S)-2-{[3-({4-[(4-chlorophényl)sulfonyl]-5-oxydo-1,2,5-oxadiazol-3-yl}oxy)propionyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1);
- Acide (2S)-2-{[3-({4-[(4-méthylphényl)sulfonyl]-5-oxydo-1,2,5-oxadiazol-3-yl}oxy)propionyl]amino}succinique - Acide (2S,3aS,7aS)-1-[(2S)-2-{[(1*S*)-1-(éthoxycarbonyl)-butyl]-amino}-propionyl]-octahydro-1*H*-indole-2-carboxylique (1:1);
- Acide (2S)-2-{[3-({4-[(4-méthylphényl)sulfonyl]-5-oxydo-1,2,5-oxadiazol-3-yl}oxy)butanoyl]amino}succinique - Acide (2S,3aS,7aS)-1-[(2S)-2-{[(1S)-1-(éthoxycarbonyl)-butyl]-amino}-propionyl]-octahydro-1*H*-indole-2-carboxylique (1:1).

La présente invention concerne également un procédé de synthèse des composés de formule (I), dans lequel le composé A est mis en réaction avec le composé B en quantité au moins égale à (n/m) équivalents du composé A.

Les composés A de formules (IIa), (IIb), (IIc), (IId), (IIe), (IIf), (IIg), (IIh), (IIi), (IIj), (IIk), (IIm) et (IIn) sont connus.

Les composés B peuvent être obtenus par des réactions classiques de la chimie organique.

A titre d'exemple, les composés B₁ et B₂ peuvent être obtenus à partir de crotonaldéhyde, que l'on transforme en 4-méthyl-1,2,5-oxadiazole 5-oxyde-3-carboxaldéhyde par réaction avec le nitrite de sodium dans l'acide acétique. L'aldéhyde ainsi obtenu est ensuite oxydé pour conduire au composé B₁ ou mis en réaction avec un dérivé protégé de l'acide triphénylphosphinométhylidène carboxylique pour conduire après déprotection au composé B₂.

Le composé B₁₂ peut être obtenu par couplage du mononitrate d'isosorbide avec l'anhydride succinique.

Les composés B de formule (III) pour lesquels Z représente le groupement Z₉ ou Z'₉ peuvent être obtenus par réaction entre le composé de formule suivante : 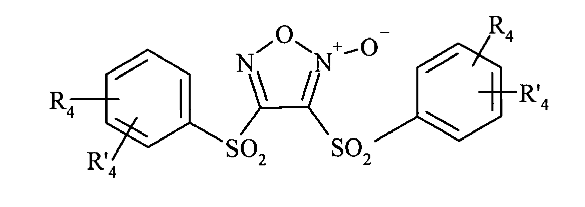 et un dérivé de l'alcool X-(Ak₁)ₓ-(Y)_{y}-(Ak₂)_{z}-OH dont les fonctions acides sont protégées, en présence de base, suivie de la déprotection des fonctions acides.

Les composés B de formule (III) pour lesquels Z représente le groupement Z₉ ou Z'₉ sont des produits nouveaux, utiles comme intermédiaires de synthèse dans l'industrie chimique ou pharmaceutique, notamment dans la synthèse des sels de formule (I), et font à ce titre partie intégrante de la présente invention.

Les composés de la présente invention, de par leurs propriétés pharmacologiques sont indiqués dans les pathologies qui nécessitent un traitement par un IEC et/ou par un donneur de NO et sont utiles dans le traitement des pathologies cardiovasculaires telles que l'hypertension artérielle (dans ses différentes formes) et ses conséquences vasculaires et rénales, l'hypertension systolique, les maladies vasculaires périphériques, l'athérosclérose, la resténose, l'insuffisance cardiaque, la thrombose et tous les événements thromboemboliques, l'angine de poitrine (stable ou instable), les accidents vasculaires cérébraux, les accidents coronariens, l'infarctus du myocarde, le remodelage vasculaire, le diabète et ses conséquences vasculaires et rénales, les complications liées aux interventions chirurgicales tels que les chirurgies cardiovasculaires et la dysfonction endothéliale.

Du fait que le NO présente également des propriétés anti-inflammatoires et anti-oxydantes, les composés de la présente invention sont utiles dans le traitement des pathologies impliquant l'inflammation et le stress oxydant.

La présente invention a également pour objet les compositions pharmaceutiques contenant comme principe actif un composé de formule (I), en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

Outre le composé de formule (I), les compositions pharmaceutiques selon l'invention contiennent un ou plusieurs excipients ou véhicules tels que des diluants, des lubrifiants, des liants, des agents de désintégration, des absorbants, des colorants, des édulcorants.

A titre d'exemple d'excipients ou véhicules, on peut citer :
◆ *pour les diluants* : le lactose, le dextrose, le sucrose, le mannitol, le sorbitol, la cellulose, la glycérine,
◆ *pour les lubrifiants* : la silice, le talc, l'acide stéarique et ses sels de magnésium et de calcium, le polyéthylène glycol,
◆ *pour les liants* : le silicate d'aluminium et de magnésium, l'amidon, la gélatine, la tragacanthe, la méthylcellulose, la carboxyméthylcellulose de sodium et la polyvinylpyrrolidone,
◆ *pour les désintégrants* : l'agar, l'acide alginique et son sel de sodium, les mélanges effervescents.

Le pourcentage de principe actif de formule (I) dans la composition pharmaceutique est préférentiellement compris entre 5% et 50% en poids.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent la présente invention. Les structures des composés décrits dans les exemples ont été déterminées selon les techniques spectrophotométriques usuelles (infrarouge, résonance magnétique nucléaire, spectrométrie de masse).

### Abréviations :

- DCC: N,N'-dicyclohexylcarbodiimide
- DIEA: N,N-diisopropyléthylamine
- DMAP: 4-diméthylaminopyridine
- DMF: diméthylformamide
- EDCI: 1-(3-diméthylaminopropyl)-3-éthylcarbodiimide (chlorhydrate)
- HOBT: 1-hydroxybenzotriazole
- THF: tétrahydrofurane
- TFA: acide trifluoroacétique

### EXEMPLE 1 : Acide (2S)-2-[(3-{[5-oxydo-4-(phénylsulfonyl)-1,2,5-oxadiazol-3-yl]-oxy}-propionyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : 3-[(5-Oxydo-4-(phénylsulfonyl)-1,2,5-oxadiazol--3-yl)-oxy]- propionate de terbutyle

A une solution refroidie à 15°C de 3,4-bis(phénylsulfonyl)-1,2,5-oxadiazole 2-oxyde (16 g- 44 mmol) et de 3-hydroxypropionate de terbutyle (10g-68 mmol) dans 200 ml de THF anhydre est additionnée en 15 minutes une solution aqueuse de NaOH à 50% (6,4g-80 mmol). Après 2h d'agitation le solvant est éliminé sous vide et le résidu repris dans 100 ml d'eau et 100 ml d'acétate d'éthyle. La phase organique est décantée, lavée avec une solution saturée de NaCl, séchée par du sulfate de sodium et évaporée.

L'huile résiduelle est purifiée par flash chromatographie, en utilisant comme éluant un mélange dichiorométhane / acétate d'éthyle (98/2) pour conduire au produit attendu.

### Stade B : Acide 3-[(5-oxydo-4-(phénylsuifonyl)-1,2,5-oxadiazol--3-yl)-oxy]-propionique (composé B₄)

14 g de composé obtenu lors du stade A, en solution dans 100 ml de dichlorométhane sont placés sous argon, additionnés de 10 ml de TFA puis agités 6 heures à température ambiante.

On élimine les solvants sous vide. Le résidu est cristallisé dans l'éther diisopropylique pour conduire au produit attendu.

*Point de fusion :* 138°C.

### Stade C : (2S)-2-[(3-{[5-Oxydo-4-(phénylsulfonyl)-1,2,5-oxadiazol-3-yl]-oxy}-propionyl)-aminoJ-succinate de diterbutyle.

Une solution du composé obtenu au stade précédent (2,7g-8 mmol), de L-aspartate de diterbutyle chlorhydrate (2,25 g- 8 mmol), EDCI (1,62 g-8 mmol), DIEA (1,32g-8mmol) et HOBT (1,1g-8mmol) dans 100 ml de DMF anhydre est agitée 72h à température ambiante sous argon. Le DMF est éliminé par distillation puis le résidu est repris dans 100 ml d'eau et 100 ml d'acétate d'éthyle. La phase organique est ensuite lavée par une solution de NaHCO₃ à 5%, séchée sur Na₂SO₄ puis évaporée à sec. On purifie le brut réactionnel par flash chromatographie en éluant par un mélange dichlorométhane/acétate d'éthyle (90:10).

### Stade D : Acide (2S)-2-[(3-{[5-oxydo-4-(phénylsulfonyl)-1,2,5-oxadiazol-3-yl]-oxy}-propionyl)-aminoJ-succinique (composé B₅).

On agite une solution du composé obtenu lors du stade précédent (4 g-7,3 mmol) dans 100ml de dichlorométhane et 15 ml de TFA pendant 6 heures sous argon. On distille les solvants à sec. On triture le résidu dans l'éther diisopropylique pendant 2h jusqu'à cristallisation. Essorer, sécher.

*Point de fusion :* 130-131°C.

### Stade E : Acide (2S)-2-[(3-{[5-oxydo-4-(phénylsulfonyl)-1,2,5-oxadiazol-3-yl]-oxy}-propionyl)-aminoJ-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

On prépare une solution de perindopril ou acide (2S,3aS,7aS)-1-[(2S)-2-{[(1S)-1-(éthoxycarbonyl)-butyl]-amino}-propionyl]-octahydro-1H-indole-2-carboxylique (368 g-1 mmol) et du composé obtenu au stade précédent (429 g-1 mmol) dans 50 ml d'eau et 50 ml d'acétonitrile. Filtrer sur Whatman et lyophiliser.

| *Microanalyse élémentaire :* | | | | |
|---|---|---|---|---|
| | *%C* | *%H* | *%N* | *%S* |
| *Calculé* | *51,19* | *5,94* | *8,78* | *4,02* |
| *Trouvé* | *51,49* | *5,79* | *8,80* | *3,70* |

### EXEMPLE 2 : Acide (2S,3aS,7aS)-1-[(2S)-2-{[(1S)-1-(éthoxycarbonyl)-butyl]-amino}-propionyl]-octahydro-1H-indole-2-carboxylique - Acide 4-méthyl-1,2,5-oxadiazole-3-carboxylique 5-oxyde (1:1)

### Stade A : 4-Méthyl-1,2,5-oxadiazole 5-oxyde-3-carboxaldéhyde

Une solution de crotonaldehyde (100g-1,42 mol) dans 200 ml d'acide acétique est refroidie à 0-5°C et additionnée en 3h30 à une solution de nitrite de sodium (340g-4.2 mol) dans 400 ml d'eau. Après 1h d'agitation, on additionne 150 ml d'eau et extrait par 6 x 100 ml de CH₂Cl₂.

Les phases organiques réunies sont lavées par 100 ml d'eau, séchées sur Na₂SO₄ et évaporées à sec.

Le résidu est purifié par flash chromatographie en utilisant comme éluant le dichlorométhane.

*Point de fusion :* 62°C.

### Stade B : Acide 4-méthyl-1,2,5-oxadiazole 5-oxyde-3-carboxylique (composé B₁)

A une solution de 7g du composé obtenu au stade A dans 400 ml d'eau sont additionnés par portions en 1h30 14,2 g de KMnO₄ (90 mmol) à une température maintenue à 20°C. On additionne ensuite 100 ml de NaOH 1M et filtre sur 200 g de célite. Le filtrat est alors acidifié par 100 ml d'HCl 1M et concentré jusqu'à un volume de 100 ml à l'évaporateur rotatif. Après extraction par 8x100 ml de dichlorométhane, les fractions organiques sont séchées sur Na₂SO₄ puis évaporées pour conduire au composé attendu.

*Point de fusion :* 92°C.

### Stade C : Acide (2S,3aS,7aS)-1-[(2S)-2-{(1S)-1-(éthoxycarbonyl)-butyl]-amino}-propionyl]-octahydro-1H-indole-2-carboxylique -Acide 4-méthyl-1, 2, 5-oxadiazole-3-carboxylique 5-oxyde (1:1)

Le composé est obtenu selon le procédé décrit au Stade E de l'Exemple 1, à partir de perindopril et du composé obtenu au stade précédent.

| *Microanalyse élémentaire :* | | | |
|---|---|---|---|
| | *%C* | *%H* | *%N* |
| *Calculé* | *53,90* | *7, 08* | *10,93* |
| *Trouvé* | *54,10* | *7, 07* | *11, 28* |

### EXEMPLE 3 : Acide (2R,3R)-2-hydroxy-3-{[5-oxydo-4-(phénylsulfonyl)-1,2,5-oxadiazol-3-yl]-oxy}-succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : (2R, 3R)-2-hydroxy-3-{[5-oxydo-4-(phénylsulfonyl)-1,2,5-oxadiazol-3-yl]-oxy}-succinate de diterbutyle

A une solution de 3,4-bis(phénylsulfonyl)-1,2,5-oxadiazole 2-oxyde (7g-19 mmol) et de tartrate de diterbutyle (5g - 19 mmol) dans 100 ml de THF anhydre, on additionne une solution de NaOH à 50% (1,2g-30 mmol) en 1/2h. Après 5h d'agitation le solvant est évaporé à sec et le résidu repris dans eau et acétate d'éthyle. La fraction organique est séchée au Na₂SO₄ et évaporée à sec puis purifiée par flash chromatographie en éluant par un mélange de dichlorométhane/éthanol (98:2), pour conduire au produit attendu sous la forme d'une huile incolore.

### Stade B : Acide (2R, 3R)-2-hydroxy-3-{[5-oxydo-4-(phénylsulfonyl)-1,2,5-oxadiazol-3-yl]-oxy}-succinique (composé B₃)

Le composé obtenu au stade précédent est agité 4h dans 50ml de dichlorométhane et 10ml de TFA.

Les solvants sont éliminés sous vide et le résidu est cristallisé dans l'éther diisopropylique. *Point de fusion :* 198-200°C.

### Stade C : Acide (2R,3R)-2-hydroxy-3-{[5-oxydo-4-(phénylsulfonyl)-1,2,5-oxadiazol-3-yl]-oxy}-succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *50,13* | *5, 70* | *7, 54* | *4, 32* |
| *Trouvé* | *49, 68* | *5, 30* | *7, 74* | *4,17* |

### EXEMPLE 4 : Acide (2S)-2-[(3-{[5-oxydo-4-(phénylsulfonyl)-1,2,5-oxadiazol-3-yl]-oxy}-propionyl)-amino]-pentanedioique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : (2S)-2-[(3-{[5-oxydo-4-(phénylsulfonyl)-1,2,5-oxadiazol-3-yl]-oxy}-propionyl)-amino]-pentanedioate de diterbutyle

Une solution préparée à partir du composé B4 obtenu au stade B de l'Exemple 1 (2,5 g-8mmol), de L-glutamate de diterbutyle-chlorhydrate (2,36g-8mmol), de EDCI (1,62g-8mmol), de DIEA (1,32ml-8mmol) et de HOBT (1,1g- 8mmol) dans 100 ml de DMF anhydre est agitée 72h à température ambiante. Le DMF est éliminé par distillation puis le résidu est repris dans 100 ml d'eau et 100 ml d'acétate d'éthyle. La phase organique est lavée par une solution de NaHCO₃ à 5% puis par de l'eau. On sèche sur Na₂SO₄ et évapore à sec. On purifie par flash chromatographie en éluant par un mélange dichlorométhane/acétate d'éthyle (90:10) pour conduire au produit attendu sous la forme d'une huile incolore.

### Stade B : Acide (2S)-2-[(3-{[5-oxydo-4-(phénylsulfonyl)-1,2,5-oxadiazol-3-yl]-oxy}-propionyl)-amino]-pentanedioique (composé B₆)

On agite une solution du composé obtenu lors du stade A (3g-5,5mmol) dans 100ml dichlorométhane et 15ml de TFA pendant 6h sous argon. On distille les solvants sous vide. Le résidu est trituré dans l'éther diisopropylique, essoré puis séché sous vide pour conduire au produit attendu sous la forme de cristaux blancs.

*Point de fusion :* 157-158°C.

### Stade C : Acide (2S)-2-[(3-{[5-oxydo-4-(phénylsulfonyl)-1,2,5-oxadiazol-3-yl)-oxy}-propionyl)-amino]-pentanedioique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *51, 78* | *6, 08* | *8, 63* | *3, 95* |
| *Trouvé* | *51, 21* | *5, 93* | *8, 69* | *3, 68* |

### EXEMPLE 5 : Acide (2E)-3-(4-méthyl-5-oxydo-1,2,5-oxadiazol-3-yl)-acrylique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : (2E)-3-(4-Méthyl-5-oxydo-1,2,5-oxadiazol-3-yl)-acrylate de terbutyle

Une solution préparée à partir du composé obtenu au stade A de l'Exemple 2 (3,9g-30mmmol) et de triphénylphosphinométhylidène carboxylate de terbutyle (13,1g-35 mmol) dans 100 ml de THF anhydre est agitée 1h à température ambiante.

On élimine le solvant sous vide puis purifie le résidu par flash chromatographie en éluant par du dichlorométhane pour conduire au produit attendu sous la forme de cristaux blancs. *Point de fusion :* 72-74°C.

### Stade B : Acide (2E)-3-(4-méthyl-5-oxydo-1,2,5-oxadiazol-3-yl)-acrylique (composé B₂)

Une solution du composé obtenu au stade précédent (6,2 g-27mmol) dans 100 ml de dichlorométhane et 10 ml de TFA est agitée 8 heures à température ambiante. Les solvants sont alors évaporés sous vide et le résidu cristallisé dans l'éther diéthylique puis essoré et séché pour conduire au produit attendu sous la forme de cristaux blancs.

*Point de fusion :* 160°C.

### Stade C : Acide (2E)-3-(4-méthyl-5-oxydo-1,2,5-oxadiazol-3-yl)-acrylique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | |
|---|---|---|---|
| | *%C* | *%H* | *%N* |
| *Calculé* | *55, 75* | *7,11* | *10, 40* |
| *Trouvé* | *55, 90* | *7, 06* | *10, 25* |

### EXEMPLE 6 : Acide 2-[(3-{[5-oxydo-4-(phénylsulfonyl)-1,2,5-oxadiazol-3-yl]-oxy}-propionyl)-amino]-éthanesulfonique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : N-{2-[(3-{[5-oxydo-4-(phénylsulfonyl)-1,2,5-oxadiazol-3-yl]-oxy}-propionyl)-oxy]}-5-norbornène-2,3-dicarboximide

Une solution du composé B4 obtenu au stade B de l'Exemple 1 (2,5 g - 5,4 mmol), de N-hydroxy-5-norbornène-2,3-dicarboximide (1,03 g - 5,4 mmol) et de DCC (1,17g-5,4mmol) dans 100 ml de THF anhydre est agitée 20 heures à température ambiante. La dicyclohexylurée formée est éliminée par filtration et le THF est éliminé par distillation sous vide.

On reprend le produit brut dans 80 ml d'acétate d'éthyle, filtre à nouveau et évapore à sec. L'ester activé ainsi obtenu est utilisé sans purification au stade B.

### Stade B : Acide 2-[(3-{[5-oxydo-4-(phénylsulfonyl)-1,2,5-oxadiazol-3-yl]-oxy}-propionyl)-amino]-éthanesulfonique (composé B₁₁)

On agite une solution de l'ester activé obtenu au stade précédent (2,56 g- 5,4 mmol), de taurine (0,81 g - 6,5 mmol), et de NaHCO₃ (0,54 g - 6,5 mmol) dans 80 ml d'eau à température ambiante pendant 48h. Les solvants sont éliminés par distillation sous vide. Les résidus sont repris dans 100 ml d'eau et 100 ml d'acétate d'éthyle. Après extraction et décantation, la phase aqueuse est éluée sur une colonne de résine DOWEX 50WX8 échangeuse de cations. Le solvant est evaporé sous vide. On purifie le résidu sur une colonne préparative HPLC phase inverse éluée par un mélange eau-acétonitrile-TFA (650:350:1). Les fractions pures sont lyophilisées.

### Stade C : Acide 2-[(3-{[5-oxydo-4-(phénylsulfonyl)-1,2,5-oxadiazol-3-yl]-oxy}-propionyl)-amino]-éthanesulfonique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *48, 66* | *6, 00* | *8, 87* | *8,12* |
| *Trouvé* | *48, 32* | *6, 32* | *8, 66* | *8, 08* |

### EXEMPLE 7 : Acide (2S,3aS,7aS)-1-[(2S)-2-{[(1S)-1-(éthoxycarbonyl)-butyl]-amino}-propionyl]-octahydro-1H-indole-2-carboxylique - Acide {[(3-{[5-oxydo-4-(phénylsulfonyl)-1,2,5-oxadiazol-3-yl]-oxy}-propionyl)-amino]-méthyl}-phosphonique (1:1)

### Stade A : Acide {[(3-{[5-oxydo-4-(phénylsulfonyl)-1,2,5-oxadiazol-3-yl]-oxy}-propionyl)-amino]-méthyl}-phosphonique (composé B₉)

Une solution préparée à partir du composé obtenu au stade A de l'Exemple 6 (2,14g-4,5 mmol), de l'acide aminométhylphosphonique (0,7g-6 mmol) et de NaHCO₃ (1g-12 mmol) dans 50 ml de dioxane et 50 ml d'eau est agitée 72h à température ambiante.

Les solvants sont alors évaporés à sec et le résidu repris dans 100 ml d'eau et 100 ml d'acétate d'éthyle et agité 1 heure. Après décantation, on purifie la phase aqueuse sur une colonne de résine DOWEX 50WX8 échangeuse de cations. Le résidu obtenu après évaporation de l'eau est cristallisé dans l'acétonitrile, essoré, puis séché sous vide pour conduire au produit attendu.

*Point de fusion :* 171-172°C.

### Stade B : Acide (2S, 3aS,7aS)-1-[(2S)-2-{[(1S)-1-(éthoxycarbonyl)-butyl]-amino}-propionyl]-octahydro-1H-indole-2-carboxylique- Acide {[(3-{[5-oxydo-4-(phénylsulfonyl)-1,2,5-oxadiazol-3-yl]-oxy}-propionyl)-amino]-méthyl}-phosphonique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *48, 00* | *5, 98* | *9, 03* | *4,13* |
| *Trouvé* | *47, 38* | *6,05* | *9, 07* | *3,97* |

### EXEMPLE 8 : Acide (2S,3aS,7aS)-1-[(2S)-2-{[(1S)-1-(éthoxycarbonyl)-butyl]-amino}-propionyl]-octahydro-1H-indole-2-carboxylique - Acide 2,2'-[(3-{[5-oxydo-4-(phénylsulfonyl)-1,2,5-oxadiazol-3-yl]-oxy}-propionyl)-imino]-diacétique (1:1)

### Stade A : 2,2'-[(3-{[5-oxydo-4-(phénylsulfonyl)-1,2,5-oxadiazol-3-yl]-oxy}-propionyl)-amino]-diacétate de terbutyle

Une solution du composé obtenu au stade B de l'Exemple 1 (3,5 g - 11 mmol), d'iminodiacétate de terbutyle (2,74 g - 11 mmol), HOBT (1,5 g - 11 mmol) et de EDCI (2,4 g - 12 mmol) dans 100 ml de DMF anhydre est agitée à température ambiante pendant 72h.

Le DMF est éliminé par distillation sous vide. Le résidu est repris dans 100 ml d'eau et 100 ml d'acétate d'éthyle. La phase organique est lavée par une solution aqueuse de NaHCO₃ à 5%, séchée sur Na₂SO₄ et évaporée à sec. On purifie le résidu par flash chromatographie en éluant par un mélange dichlorométhane-acétate d'éthyle (95:5) pour conduire au produit attendu sous la forme d'une huile incolore.

### Stade B : Acide 2,2'-[(3-{[5-oxydo-4-(phénylsulfonyl)-1,2,5-oxadiazol-3-yl]-oxy}-propionyl)-aminoJ-diacétique (composé B₈)

Une solution du composé obtenu au stade précédent (800 mg - 1,5 mmol) dans 50 ml de dichlorométhane et 5 ml de TFA est agitée 6h à température ambiante. Les solvants sont alors évaporés sous vide. Le résidu est cristallisé dans l'éther diisopropylique, essoré, puis séché sous vide de pompe pour conduire au produit attendu sous la forme de cristaux blancs.

*Point de fusion :* 154°C (décomposition).

### Stade C : Acide (2S,3aS,7aS)-1-[(2S)-2-{[(1S)-1-(éthoxycarbonyl)-butyl]-amino}-propionyl]-octahydro-1H-indole-2-carboxylique -Acide 2,2'-[(3-{[5-oxydo-4-(phénylsulfonyl)-1,2,5-oxadiazol-3-yl]-oxy}-propionyl)-imino]-diacétique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *51,19* | *5,94* | *8,78* | *4, 02* |
| *Trouvé* | *51,41* | *5,93* | *8,79* | *3,56* |

### EXEMPLE 9 : Acide (2S,3aS,7aS)-1-[(2S)-2-{[(1S)-1-(éthoxycarbonyl)-butyl]-amino}-propionyl]-octahydro-1H-indole-2-carboxylique - Acide {2-[(3-{[5-oxydo-4-(phénylsulfonyl)-1,2,5-oxadiazol-3-yl]-oxy}-propionyl)-amino]-éthyl}-phosphonique (1:1)

### Stade A : Acide {[(3-{[5-oxydo-4-(phénylsulfonyl)-1,2,5-oxadiazol-3-yl]-oxy}-propionyl)-amino]-éthyl}-phosphonique (composé B₇)

Le composé est obtenu selon le procédé décrit au Stade A de l'Exemple 7, en remplaçant l'acide aminométhylphosphonique par l'acide aminoéthylphosphonique.

*Point de fusion : 162°C.*

### Stade B : Acide (2S,3aS,7aS)-1-[(2S)-2-{[(1S)-1-(éthoxycarbonyl)-butyl]-amino}-propionyl]-octahydro-1H-indole-2-carboxylique - Acide {2-[(3-{[5-oxydo-4-(phénylsulfonyl)-1,2,5-oxadiazol-3-yl]-oxy}-propionyl)-amino]-éthyl}-phosphonique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *48, 66* | *6,13* | *8,87* | *4, 06* |
| *Trouvé* | *48,81* | *6, 06* | *8,90* | *4,42* |

### EXEMPLE 10 : Acide (2S,3aS,7aS)-1-[(2S)-2-{[(1S)-1-(éthoxycarbonyl)-butyl]-amino}-propionyl]-octahydro-1H-indole-2-carboxylique - Acide (2-{[5-oxydo-4-(phénylsulfonyl)-1,2,5-oxadiazol-3-yl]-oxy}-éthyl)-phosphonique (1:1)

### Stade A : (2-{[5-Oxydo-4-(phénylsulfonyl)-1,2,5-oxadiazol-3-yl]-oxy}-éthyl phosphonate de diméthyle

A une solution de 3,4-bis(phénylsulfonyl)-1,2,5-oxadiazole 2-oxyde (7,5 g - 20,7 mmol ), et de 2-hydroxy-éthyl phosphonate de diméthyle (4,5 g - 31 mmol) dans 100 ml de THF, on additionne une solution de NaOH à 50% dans l'eau (1,5 g - 32 mmol) en 1/2h. Après 1h d'agitation on élimine le solvant sous vide et on reprend le résidu par de l'eau (100ml) et de l'acétate d'éthyle (100ml). La phase organique est séchée sur Na₂SO₄ puis évaporée à sec. On purifie le résidu par flash chromatographie en éluant par un mélange dichlorométhane/éthanol (95 /5) pour conduire au produit attendu sous la forme d'une huile incolore.

### Stade B : Acide (2-{[5-oxydo-4-(phénylsulfonyl)-1,2,5-oxadiazol-3-yl]-oxy}-éthyl)-phosphonique (composé B₁₀)

A une solution du composé obtenu au stade précédent (1,8 g - 4 mmol) dans 50 ml de dioxane, on additionne 2,9 ml de bromure de triméthylsilyle (4 eq.) puis chauffe le milieu réactionnel pendant 4 heures à 60°C.

Les solvants sont évaporés à sec. Le résidu est purifié sur une colonne Biogel éluée par un mélange eau / acétonitrile (1 /1). Les fractions contenant le produit pur sont lyophilisées.

### Stade C : Acide (2S,3aS,7aS)-1-[(2S)-2-{[(1S)-1-(éthoxycarbonyl)-butyl]-amino}-propionyl]-octahydro-1H-indole-2-carboxylique - Acide (2-{[5-oxydo-4-(phénylsulfonyl)-1,2,5-oxadiazol-3-yl]-oxy}-éthyl)-phosphonique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *48,46* | *6, 03* | *7,80* | *4,46* |
| *Trouvé* | *48,53* | *5,97* | *7,95* | *4, 61* |

### EXEMPLE 11 : Acide (2S,3aS,7aS)-1-[(2S)-2-{[(1S)-1-(éthoxycarbonyl)-butyl]-amino}-propionyl]-octahydro-1H-indole-2-carboxylique - Succinate de mono-(6-nitrooxy-hexahydro-furo[3.2-b] furan-3-yle) (1:1)

### Stade A : Succinate de mono-(6-nitrooxy-hexahydro-furo[3.2-b]furan-3-yl) (composé B₁₂)

Une solution de mononitrate d'isosorbide (5,73g -30mmol), d'anhydride succinique (3.0g - 30mmol) et 100 mg de DMAP dans 100 ml d'acétonitrile est portée à reflux pendant 20 h. Le solvant est alors éliminé par évaporation. On purifie le produit brut par flash chromatographie en utilisant comme éluant un mélange dichlorométhane / éthanol (98:2). Le produit obtenu est recristallisé dans l'éther diisopropylique pour conduire au produit attendu sous la forme d'un solide blanc.

*Point de fusion :* 65°C.

### Stade B : Acide (2S,3aS,7aS)-1-[(2S)-2-{[(1S)-1-(éthoxycarbonyl)-butyl]-amino}-propionyl]-octahydro-1H-indole-2-carboxylique - Succinate de mono-(6-nitrooxy-hexahydro-furo[3.2-b]furan-3-yle) (1:1)

Le composé est obtenu selon le procédé décrit au Stade E de l'Exemple 1, à partir de perindopril et du composé obtenu au stade précédent.

| *Microanalyse élémentaire :* | | | |
|---|---|---|---|
| | *%C* | *%H* | *%N* |
| *Calculé* | *52,80* | *6,88* | *6,37* |
| *Trouvé* | *52, 66* | *6,87* | *6, 65* |

### EXEMPLE 12 : Acide (2S,3aS,7aS)-1-[(2S)-2-{[(1S)-1-(éthoxycarbonyl)-butyl]-amino}-propionyl]-octahydro-1H-indole-2-carboxylique - Acide N-(3-{[5-oxydo-4-(phénylsulfonyl-1,2,5-oxadiazol-3-yl]-oxy}-propionyl)-glycine (1:1)

### Stade A : N-(3-{[5-oxydo-4-(phénysulfonyl-1,2,5-oxadiazol-3-yl]-oxy}-propionyl)-glycinate de terbutyle

Une solution du composé obtenu au stade B de l'Exemple 1 (1,5g-4 mmol), de glycinate de terbutyle (0,6g-4 mmol), EDCI (0,81g-4 mmol) et de HOBT (0,57g-4mmol) dans 100 ml de DMF anhydre est agitée 24h à température ambiante sous argon. Le DMF est alors éliminé par distillation et le résidu repris dans 100 ml d'eau et 100 ml d'acétate d'éthyle. La phase organique est lavée par une solution de NaHCO₃ à 5% dans l'eau, séchée sur Na₂SO₄ puis évaporée à sec. On purifie par flash chromatographie en éluant par un mélange dichlorométhane -acétate d'éthyle (95 :5) pour conduire au produit attendu sous la forme d'une huile incolore.

### Stade B : Acide N-(3-{[5-oxydo-4-(phénylsulfonyl-1,2,5-oxadiazol-3-yl]-oxy}-propionyl)-glycine (composé B₁₃)

Une solution du composé obtenu au stade précédent (1,7g- 4mmol) dans 100 ml de dichlorométhane et 10 ml de TFA est agitée 6h à température ambiante.

Les solvants sont alors éliminés sous vide et le résidu trituré dans l'éther diéthylique pendant 2 heures jusqu'à cristallisation. Essorer, sécher sous vide de pompe.

*Point de fusion* : 177°C.

### Stade C : Acide (2S,3aS,7aS)-1-[(2S)-2-{[(1S)-1-(éthoxycarbonyl)-butyl]-amino}-propionyl]-octahydro-1H-indole-2-carboxylique -Acide N-(3-{[5-oxydo-4-(phénylsulfonyl-1,2,5-oxadiazol-3-yl]-oxy}-propionyl)-glycine (1:1)

Le composé est obtenu selon le procédé décrit au Stade E de l'Exemple 1, à partir de perindopril et du composé obtenu au stade précédent.

### EXEMPLE 13 : Acide (2S)-2-[(4-{[5-oxydo-4-(phénylsulfonyl)-1,2,5-oxadiazol-3-yl]-oxy}-butanoyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide 4-[(5-oxydo-4-(phénylsulfonyl)-1,2,5-oxadiazol--3-yl)-oxy]-butanoïque (composé B₁₆)

Le composé attendu est obtenu selon le procédé décrit aux stades A et B de l'Exemple 1, en remplaçant au Stade A le 3-hydroxypropionate de terbutyle par le 4-hydroxybutanoate de terbutyle.

*Point de fusion* : 130-131°C

### Stade B : Acide (2S)-2-[(4-{[5-oxydo-4-(phénylsulfonyl)-1,2,5-oxadiazol-3-yl)-oxy}-butanoyl)-amino]-succinique (composé B₁₄).

Le composé attendu est obtenu selon le procédé décrit aux stades C et D de l'Exemple 1, à partir du composé obtenu au stade précédent.

### Stade C : Acide (2S)-2-[(4-{[5-oxydo-4-(phénylsulfonyl)-1,2,5-oxadiazol-3-yl]-oxy}-butanoyl)-amino]-succinique- Acide (2S,3aS,7aS)-1-[(2S)-2-{[(1S)-1-(éthoxycarbonyl)-butyl]-amino}-propionyl}-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *51,78* | *6, 08* | *8, 63* | *3,95* |
| *Trouvé* | *52,52* | *6,05* | *8,82* | *3,47* |

### EXEMPLE 14 : Acide {[5-oxydo-4-(phénylsulfonyl)-1,2,5-oxadiazol-3-yl]-oxy}-acétique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide {[5-oxydo-4-(phénylsulfonyl)-1,2,5-oxadiazol-3-yl]oxy}acétique (composé B₁₅)

Le composé attendu est obtenu selon le procédé décrit aux stades A et B de l'Exemple 1, en remplaçant au Stade A le 3-hydroxypropionate de terbutyle par l'hydroxyacétate de terbutyle.

### Stade B : Acide {[5-oxydo-4-(phénylsuifonyl)-1,2,5-oxadiazol-3-yl]-oxy}-acétique Acide (2S,3aS,7aS)-1-[(2S)-2-{[(1S)-1-(éthoxycarbonyl)-butyl]-amino}propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *52, 09* | *6, 03* | *8,38* | *4,80* |
| *Trouvé* | *51,76* | *5,84* | *8,50* | *4,97* |

### EXEMPLE 15 : Acide 3-[(5-oxydo-4-(phénylsulfonyl)-1,2,5-oxadiazol-3-yl)-oxy]-propionique- Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade B de l'Exemple 1.

### EXEMPLE 16 : Acide 4-[(5-oxydo-4-(phénylsulfonyl)-1,2,5-oxadiazol-3-yl)-oxy]-butanoïque - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade A de l'Exemple 13.

### EXEMPLE 17 : Acide 3-({4-[(4-chlorophényl)sulfonyl]-5-oxydo-1,2,5-oxadiazol-3-yl}oxy)-propionique- Acide (2S,3aS,7aS)-1-[(25)-2-{[(1S)-1-(éthoxycarbonyl)-butyl]-amino}-propionyl]-octahydro-1H-indole-2-carboxylique (1:1)

### Stade A : Acide 3-({4-[(4-chlorophényl)suljonyl}-5-oxydo-1,2,5-oxadiazol-3-yl}oxy)-propionique (composé B₁₇)

Le composé attendu est obtenu selon le procédé décrit aux stade A et B de l'Exemple 1 en remplaçant au stade A le 3,4-bis(phénylsulfonyl)-1,2,5-oxadiazole 2-oxyde par le 3,4-bis[(4-chlorophényl)-sulfonyl]-1,2,5-oxadiazole 2-oxyde.

### Stade B : Acide 3-({4-[(4-chlorophényl)sulfonyl]-5-oxydo-1,2,5-oxadiazol-3-yl}oxy)-propionique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

### EXEMPLE 18 : Acide (2S)-2-{[3-({4-[(4-chlorophényl)sulfonyl]-5-oxydo-1,2,5-oxadiazol-3-yl}oxy)propionyl]amino}succinique- Acide (2S,3aS,7aS)-1-[(2S)-2-1[(1S)-1-(éthoxycarbonyl)-butyl]-amino}-propionyl]-octahydro-1H-indole-2-carboxylique (1:1)

### Stade A : Acide (2S)-2-{[3-({4-[(4-chlorophényl)sulfonyl]-5-oxydo-1,2,5-oxadiazol-3-yl}oxy)propionyl]amino}succinique (composé B₁₈)

Le composé attendu est obtenu selon le procédé décrit aux stades C et D de l'Exemple 1 à partir du composé obtenu au stade A de l'Exemple 17.

### Stade B : Acide (2S)- 2-{[3-({4-[(4-chlorophényl)sulfonyl]-5-oxydo-1,2,5-oxadiazol-3-yl}oxy)propionyl]amino}succinique - Acide (2S,3aS, 7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | | |
|---|---|---|---|---|---|
| | *%C* | *% H* | *% N* | *%S* | *%Cl* |
| *Calculé* | *49,07* | *5,57* | *8,41* | *3,85* | *4, 26* |
| *Trouvé* | *49,12* | *5,82* | *8, 27* | *3,70* | *4,12* |

### EXEMPLE 19 : Acide (2S)-2-[(3-{[5-oxydo-4-(phénylsulfonyl)-1,2,5-oxadiazol-3-yl]-oxy}-acétyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-[(3-{[5-oxydo-4-(phénylsulfonyl)-1,2,5-oxadiazol-3-yl]-oxy}-acétyl)-amino]-succinique (composé B₂₄).

Le composé attendu est obtenu selon le procédé décrit aux stades A à D de l'Exemple 1 en remplaçant au stade A le 3-hydroxypropionate de terbutyle par l'hydroxyacétate de terbutyle.

### Stade B : Acide (2S)-2-[(3-{[5-oxydo-4-(phénylsulfonyl)-1,2,5-oxadiazol-3-yl)-oxy}-acétyl)-amino]-succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *50,57* | *5,79* | *8,94* | *4, 09* |
| *Trouvé* | *50, 71* | *5,79* | *9, 04* | *3,82* |

### EXEMPLE 20 : Acide (2S)-2-[(2-{[5-oxydo-4-(phénylsulfonyl)-1,2,5-oxadiazol-3-yl]oxy}éthyl)amino]succinique : Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : (2S)-2-[(2-Hydroxyéthyl)amino]succinate de diterbutyle

Une solution de L-aspartate de diterbutyle (5,62 g - 20 mmol), de 2-bromoéthanol (2,84 mL - 40 mmol) et de K₂CO₃ (5,6 g - 40 mmol) dans 100 mL d'acétonitrile est portée à reflux pendant 16 heures. Les sels minéraux sont filtrés et le solvant éliminé par distillation sous vide. On purifie le produit brut ainsi obtenu par flash chromatographie sur gel de silice en utilisant comme éluant un mélange dichlorométhane - éthanol (95 :5). On obtient le produit attendu sous la forme d'une huile incolore.

### Stade B : Acide (2S)-2-[(2-{[5-oxydo-4-(phénylsulfonyl)-1,2,5-oxadiazol-3-yl]oxy}éthyl)amino]succinique (composé B₂₅)

Le composé est obtenu selon le procédé décrit aux stades A et B de l'Exemple 1, en remplaçant le 3-hydroxypropionate de terbutyle par le composé obtenu au stade précédent.

*Point de fusion : 160-161°C.*

### Stade C : Acide (2S)-2-[(2-{[5-oxydo-4-(phénylsulfonyl)-1,2,5-oxadiazol-3-yl]oxy}éthyl)amino]succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *51,49* | *6,15* | *9,10* | *4,17* |
| *Trouvé* | *51,05* | *5,94* | *9, 20* | *4, 06* |

### EXEMPLE 21 : Acide (2S)-2-{[3-({4-[(4-méthylphényl)sulfonyl]-5-oxydo-1,2,5 oxadiazol-3-yl}oxy)propionyl]amino}succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-{[3-({4-[(4-méthylphényl)sulfonyl]-5-oxydo-1,2,5-oxadiazol-3-yl}oxy)propionyl]amino}succinique (composé B₂₁)

Le composé est obtenu selon le procédé décrit aux Stades A à D de l'Exemple 1, en remplaçant au Stade A le 3,4-bis(phénylsulfonyl)-1,2,5-oxadiazole 2-oxyde par le 3,4-bis[(4-méthylphényl)sulfonyl]-1,2,5-oxadiazole 2-oxyde.

*Point de fusion : 167-168°C.*

### Stade B : Acide (2S)-2-{[3-({4-[(4-méthylphényl)sulfonyl}-5-oxydo-1,2,5-oxadiazol-3-yl}oxy)propionyl]amino}succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *% C* | *%H* | *%N* | *%S* |
| *Calculé* | *51,78* | *6, 08* | *8, 63* | *3,95* |
| *Trouvé* | *51,92* | *5,96* | *8,45* | *3,96* |

### EXEMPLE 22 : Acide (2S)-2-{[3-({4-[(4-chlorophényl)sulfonyl]-5-oxydo-1,2,5-oxadiazol-3-yl}oxy)butanoyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-{[3-({4-[(4-chlorophényl)sulfonyl]-5-oxydo-1,2,5-oxadiazol-3-yl}oxy)butanoyl]amino}succinique (composé B₂₆)

Le composé attendu est obtenu selon le procédé décrit aux stades A à D de l'Exemple 1, en remplaçant au Stade A le 3-hydroxypropionate de tert-butyle par le 4-hydroxybutanoate de tert-butyle, et le 3,4-bis(phénylsulfonyl)-1,2,5-oxadiazole 2-oxyde par le 3,4-bis[(4-chlorophényl)-sulfonyl]-1,2,5-oxadiazole 2-oxyde.

*Point de fusion : 145-146°C.*

### Stade B : Acide (2S)-2-{[3-({4-[(4-chlorophényl)sulfonyl]-5-oxydo-1,2,5-oxadiazol-3-yl}oxy)butanoyl]amino}succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | | |
|---|---|---|---|---|---|
| | *% C* | *%H* | *%N* | *%S* | *%Cl* |
| *Calculé* | *49, 67* | *5,72* | *8, 28* | *3,79* | *4,19* |
| *Trouvé* | *49,44* | *5,78* | *8, 20* | *3,70* | *4,10* |

### EXEMPLE 23 : Acide (2S)- 2-{[3-({4-[(4-méthylphényl)sulfonyl]-5-oxydo-1,2,5-oxadiazol-3-yl}oxy)butanoyl]amino}succinique- Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide (2S)-2-{[3-({4-[(4-méthylphényl)sulfonyl}-5-oxydo-1,2,5-oxadiazol-3-yl}oxy)butanoyl]amino}succinique (composé B₂₇)

Le composé attendu est obtenu selon le procédé décrit aux stades A à D de l'Exemple 1, en remplaçant au Stade A le 3-hydroxypropionate de tert-butyle par le 4-hydroxybutanoate de tert-butyle, et le 3,4-bis(phénylsulfonyl)-1,2,5-oxadiazole 2-oxyde par le 3,4-bis[(4-méthylphényl)-sulfonyl]-1,2,5-oxadiazole 2-oxyde.

*Point de fusion : 147-148°C.*

### Stade B : Acide (2S)-2-{[3-({4-[(4-méthylphényl)suljonyl]-5-oxydo-1,2, 5-oxadiazol-3-yl}oxy)butanoyl]amino}succinique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *52,36* | *6,22* | *8,48* | *3,88* |
| *Trouvé* | *52, 66* | *6,15* | *8, 67* | *4,16* |

### EXEMPLE 24 : Acide 2-[(3-{[5-oxydo-4-(phénylsulfonyl)-1,2,5-oxadiazol-3-yl]-oxy}-butanoyl)-amino]-éthanesulfonique - Acide (2S,3aS,7aS)-1-[(2S)-2-{[(1S)-1-(éthoxycarbonyl)-butyl]-amino}-propionyl]-octahydro-1H-indole-2-carboxylique (1:1)

### Stade A : Acide 2-[(3-{[5-oxydo-4-(phénylsulfonyl)-1,2,5-oxadiazol-3-yl]-oxy}-butanoyl)-amino]-éthanesulfonique (composé B₂₈)

Le composé attendu est obtenu selon le procédé décrit aux Stades A et B de l'Exemple 6, en remplaçant au Stade A le composé B₄ par le composé B₁₆ obtenu au Stade A de l'Exemple 13.

*Point de fusion : 131-132°C.*

### Stade B : Acide 2-[(3-{[5-oxydo-4-(phénylsulfonyl)-1,2,5-oxadiazol-3-yl]-oxy}-butanoyl)-amino]-éthanesulfonique - Acide (2S,3aS,7aS)-1-[(2S)-2-{[(1S)-1-(éthoxycarbonyl)-butyl]-amino}-propionyl]-octahydro-1H-indole-2-carboxylique (1:1)

Le composé est obtenu selon le procédé décrit au Stade E de l'Exemple 1, à partir de perindopril et du composé obtenu au stade précédent.

| *Microanalyse élémentaire :* | | | | |
|---|---|---|---|---|
| | *%C* | *%H* | *%N* | *%S* |
| *Calculé* | *49,30* | *6,14* | *8,71* | *7,98* |
| *Trouvé* | *49,15* | *6,12* | *8,82* | *8, 22* |

### EXEMPLE 25 : N,N-Bis(2-sulfoéthyl)-4-{[5-oxydo-4-(phénylsulfonyl)-1,2,5-oxadiazol-3-yl]oxy}butanamide - Acide (2S,3aS,7aS)-1-[(2S)-2-{[(1S)-1-(éthoxycarbonyl)-butyl]-amino}-propionyl]-octabydro-1H-indole-2-carboxylique (1:1)

### Stade A : N-{2-[(3-{[5-oxydo-4-(phénylsulfonyl)-1,2,5-oxadiazol-3-yl]-oxy}-butanoyl)-oxy]}-5-norbornène-2,3-dicarboximide

Le composé attendu est obtenu selon le procédé décrit au Stade A de l'Exemple 6, en remplaçant le composé B₄ par le composé B₁₆ obtenu au Stade A de l'Exemple 13.

### Stade B : N,N-Bis(2-sulfoéthyl)-4-{[5-oxydo-4-(phénylsulfonyl)-1,2,5-oxadiazol-3-yl]oxy}butanamide (composé B₂₉)

Le composé attendu est obtenu selon le procédé décrit au Stade B de l'Exemple 6, à partir de ditaurine (préparée selon DE 10033580) et du composé obtenu au stade précédent.

### Stade C : N,N-Bis(2-sulfoéthyl)-4-{[5-oxydo-4-(phénylsulfonyl)-1,2,5-oxadiazol-3-yl]oxy}butanamide - Acide (2S,3aS,7aS)-1-[(2S)-2-{[(1S)-1-(éthoxycarbonyl)-butyl]-amino}-propionyl]-octahydro-1H-indole-2-carboxylique(1:1)

Le composé est obtenu selon le procédé décrit au Stade E de l'Exemple 1, à partir de perindopril et du composé obtenu au stade précédent.

### ETUDE PHARMACOLOGIQUE DES PRODUITS DE L'INYENTION

### EXEMPLE 26 : Activité donneur de NO

### In vitro

Les anneaux d'aorte sans endothélium sont utilisés. Après une première contraction induite par du KCl 60 mM pour caractériser la sensibilité de l'anneau et un lavage, une contraction stable est induite par de la noradrenaline (0,1-0,3 µM) en présence ou non de l'inhibiteur de la guanylate cyclase, l'ODQ (10 µM). Une gamme de concentrations cumulées est réalisée et l'activité du produit à tester est calculée par une IC₅₀ (dose inhibant de 50 % l'effet maximal).

Résultats : les composés selon l'invention présentent un effet relaxant tout à fait significatif ;
à titre d'exemple, les composés des Exemples 1 et 7 ont des IC₅₀ de 0,048 et 0,047 µM respectivement.

### EXEMPLE 27 : Activité inhibitrice de l'enzyme de conversion de l'angiotensine I

### In vivo

Les rats sont anesthésiés au pentobarbital, placés en respiration artificielle, la pression artérielle moyenne est mesurée par un cathéter placé dans l'artère fémorale et relié à un capteur de pression. Les nerfs vagues sont sectionnés et la mécamilamine (bloqueur des ganglions sympathiques) est injectée i.v. à 1.5 mg/kg. Après stabilisation, l'angiotensine I est injectée i.v. à 1.5 µg/kg.

La réponse hypertensive maximale à l'angiotensine I est mesurée sur des rats ayant reçu 60 minutes avant l'anesthésie, par voie orale soit de la gomme du Sénégal seule (groupe contrôle), soit la molécule testée dans la gomme du Sénégal. L'effet inhibiteur est déterminé en % de la réponse à l'angiotensine I versus le groupe contrôle.

Résultats : les composés selon l'invention présentent un effet inhibiteur tout à fait significatif ;
à titre d'exemple, les composés des Exemples 1 et 7, injectées à la dose de 0,1 mg/kg provoquent des inhibitions de l'angiotensine I de 85 et 88 % respectivement.

### EXEMPLE 28 : Composition pharmaceutique

| Formule de préparation pour 1000 comprimés dosés à 100 mg | |
|---|---|
| Composé de l'un des Exemples 1 à 25 | 100 g |
| Hydroxypropycellulose | 2 g |
| Amidon de blé | 10 g |
| Lactose | 100 g |
| Stéarate de magnésium | 3 g |
| Talc | 3 g |

## Revendications

1. Composés de formule (I) :
(A)m • (B)ₙ (I)
dans laquelle A représente un composé inhibiteur de l'enzyme de conversion de l'angiotensine comportant au moins une fonction basique salifiable, de formule (II) : 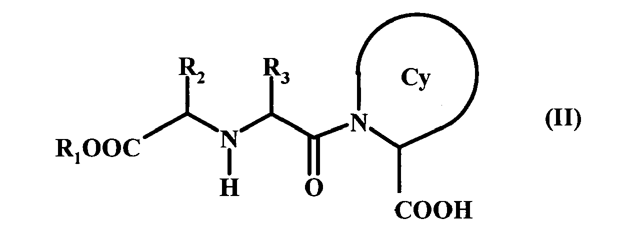 dans laquelle :
- R₁ représente un atome d'hydrogène ou un groupement alkyle C₁-C₆ linéaire ou ramifié,
- R₂ représente un groupement alkyle C₁-C₆ linéaire ou ramifié ou aryl-(C₁-C₆) alkyle dans lequel le groupement alkyle est linéaire ou ramifié,
- R₃ représente un groupement alkyle C₁-C₆ linéaire ou ramifié ou amino-(C₁-C₆) alkyle dans lequel le groupement alkyle est linéaire ou ramifié,
- 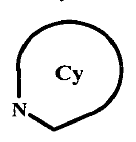 représente un système mono- ou bicyclique azoté comportant de 3 à 12 atomes de carbone et pouvant comporter un ou plusieurs hétéroatomes supplémentaires choisis parmi N, O et S, ledit système étant éventuellement substitué par un ou plusieurs groupement choisis parmi alkyle C₁-C₆ linéaire ou ramifié, alkoxy C₁-C₆ linéaire ou ramifié, oxo, carboxy, aryle, cycloalkyle C₃-C₈, hétéroaryle, carboxy-(C₁-C₆) alkyle dans lequel le groupement alkyle est linéaire ou ramifié, hydroxy, étant entendu que lorsque le système azoté est bicyclique, il peut être fusionné, spiranique ou ponté,
B représente un composé comportant au moins une fonction acide salifiable et au moins un groupement donneur de NO,
m représente le nombre de fonctions acides salifiées de B et n représente le nombre de fonctions basiques salifiées de A,
étant entendu que la ou les liaisons entre A et B sont de type ionique.

2. Composés de formule (I) selon la revendication 1, où 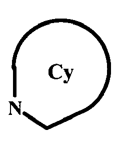 représente un cycle pyrrolidine, perhydroindole, octahydro- cyclopenta[b]pyrrole, imidazolidine ou tétrahydroisoquinoléine.

3. Composés de formule (I) selon la revendication 1, où A représente l'énalapril, l'énalaprilate, le lisinopril, le perindopril, le perindoprilate, le ramipril, le spirapril, le trandolapril, le trandolaprilate, l'imidapril, le moexipril, le quinapril ou le ramiprilate.

4. Composés de formule (I) selon la revendication 1, choisi parmi les composés suivants : 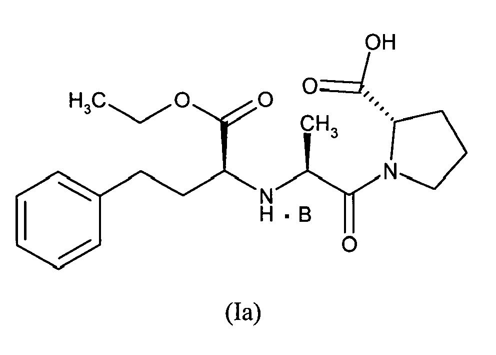 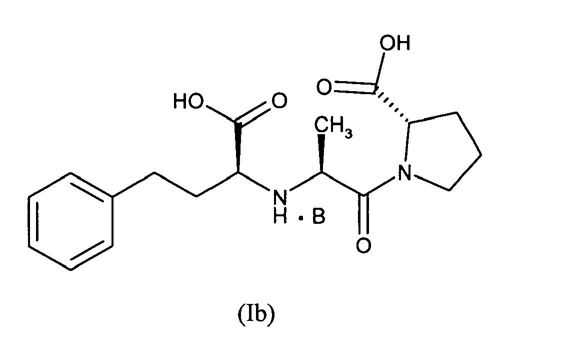 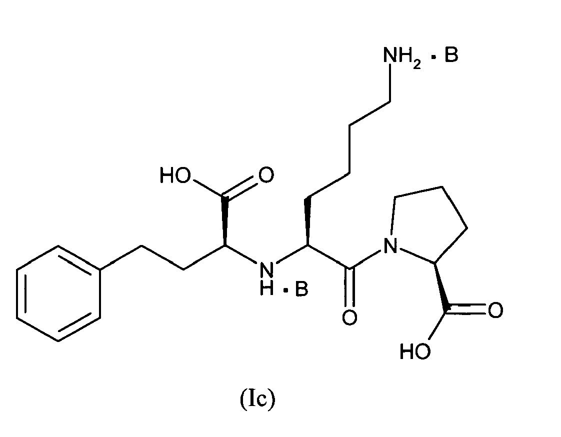 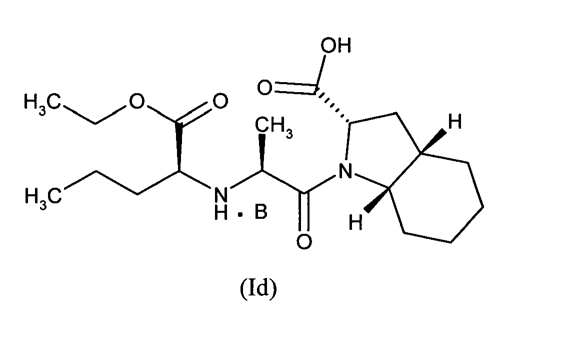 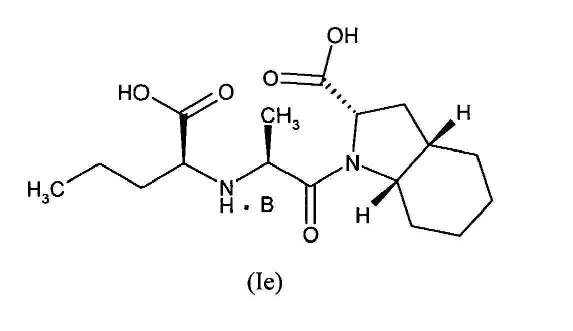 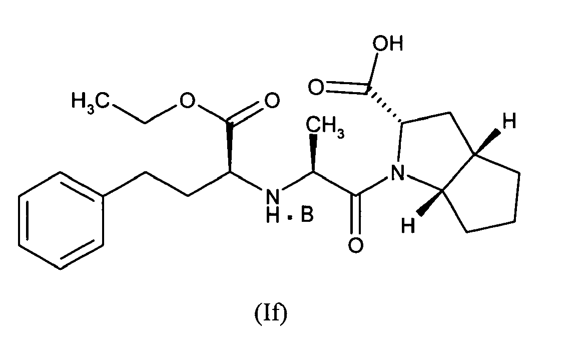 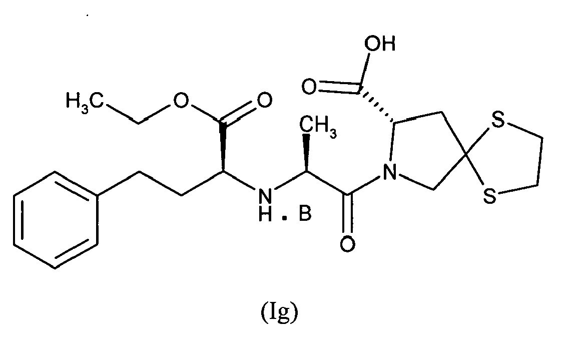 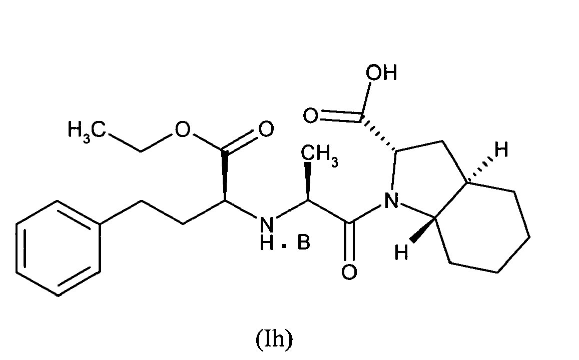 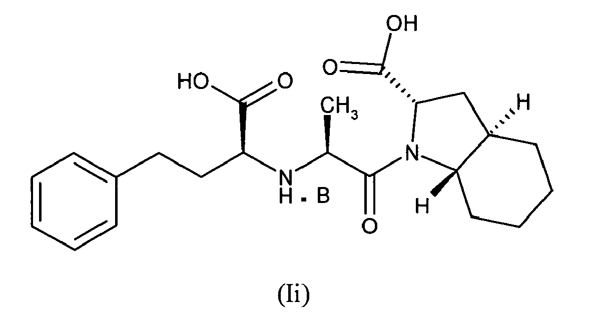 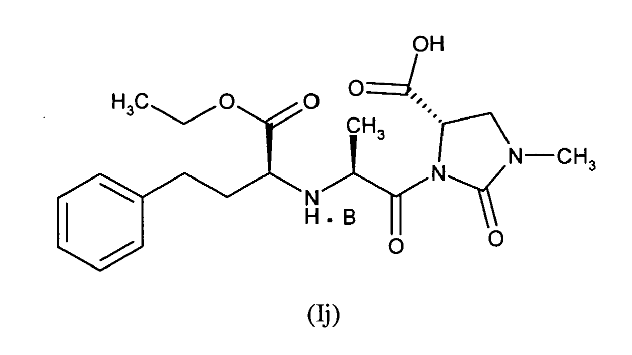 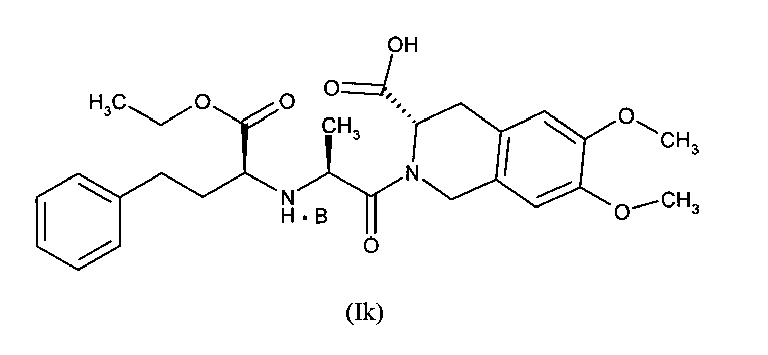 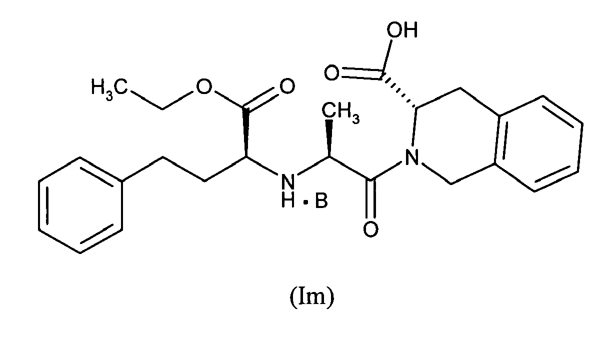 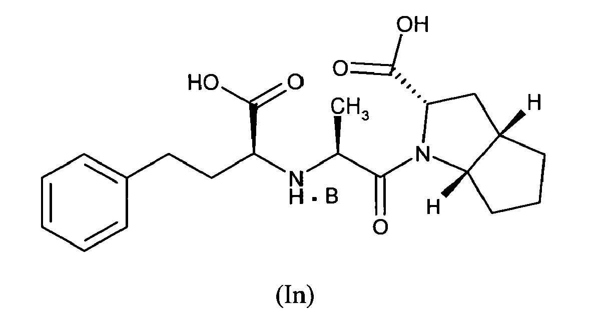 où B est tel que défini dans la revendication 1.

5. Composés de formule (I) selon l'une quelconque des revendications 1 à 4, où B appartient à la formule (III) suivante :
X-(Akₗ)ₓ-(Y)_{y}-(Ak₂)_{z}-Z (III)
dans laquelle :
- X représente un groupement CO₂H, SO₃H ou P(O)(OH)₂,
- Ak₁ et Ak₂, identiques ou différents, représentent chacun un groupement alkylène C₁-C₈ linéaire ou ramifié, saturé ou insaturé, dans lequel un ou plusieurs des atomes de carbone peuvent être remplacés par un atome d'oxygène, de soufre, d'azote ou un groupement SO₂, ledit groupement alkylène étant éventuellement substitué par un ou plusieurs groupements choisis parmi CO₂H, SO₃H, hydroxy et amino,
- x, y et z, identiques ou différents, représentent chacun 0 ou 1,
- Y représente CO ou CONH,
- Z représente un groupement donneur de NO.

6. Composés de formule (I) selon la revendication 5, où le composé de formule (III) est choisi parmi les composés de formules suivantes :
HO₂C-(Ak₁)ₓ-Z (IIIa)
HO₂C-CH=CH-(Ak₃)ₓ-Z (IIIb) 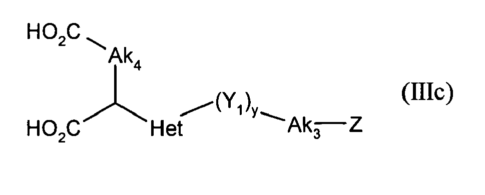 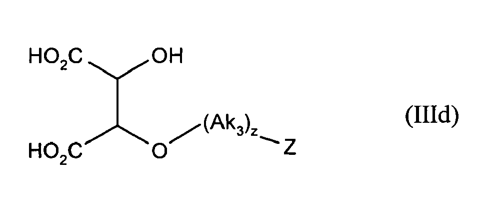 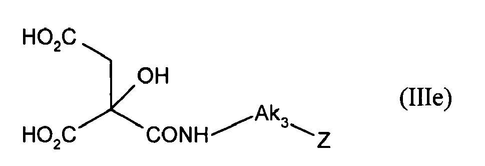 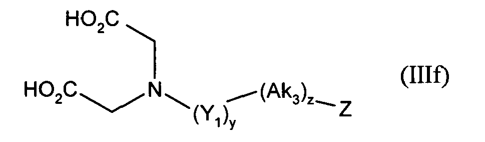 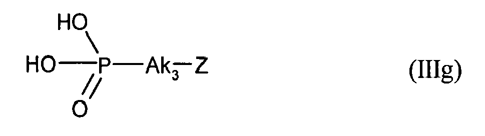 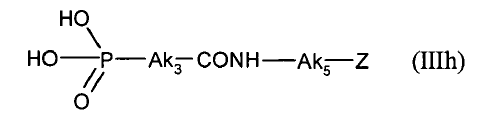
HO₃S-Ak₆-CONH-Ak₅-Z (IIIi) 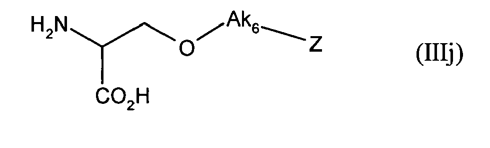 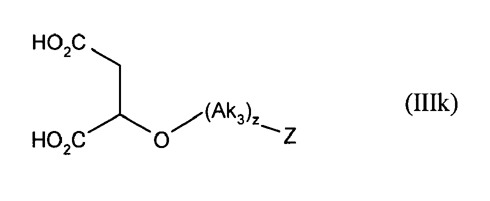
dans lesquelles :
Ak₁, x, y, z et Z sont tels que définis dans la revendication 5,
Ak₃ et Ak₅, identiques ou différents, représentent chacun un groupement alkylène C₁-C₆ linéaire ou ramifié saturé dans lequel un ou plusieurs des atomes de carbone peuvent être remplacés par un atome d'oxygène, de soufre, d'azote ou un groupement SO₂,
Ak₄ représente un groupement alkylène C₁-C₃ linéaire ou ramifié saturé dans lequel un des atomes de carbone peut être remplacé par un atome d'oxygène, de soufre, d'azote ou un groupement SO₂,
Ak₆ représente un groupement alkylène C₂-C₅ linéaire ou ramifié saturé dans lequel un des atomes de carbone peut être remplacé par un atome d'oxygène, de soufre, d'azote ou un groupement SO₂,
Het représente O ou NH,
et Y₁ représente un groupement carbonyle.

7. Composés de formule (I) selon l'une quelconque des revendications 5 ou 6, où Z est choisi parmi les groupements suivants : 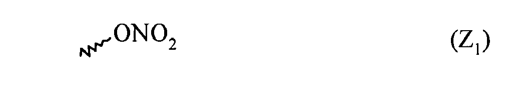 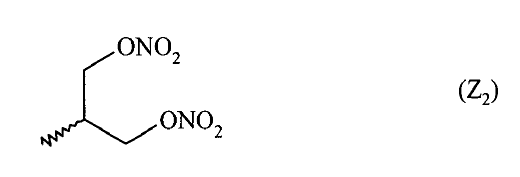 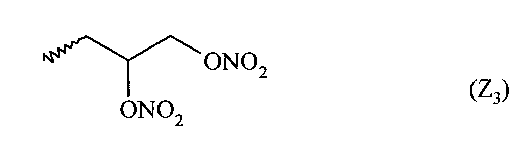 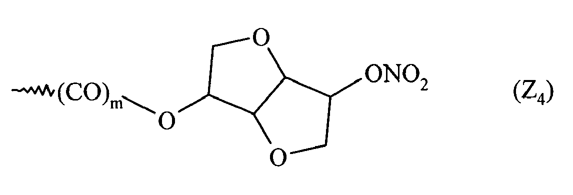 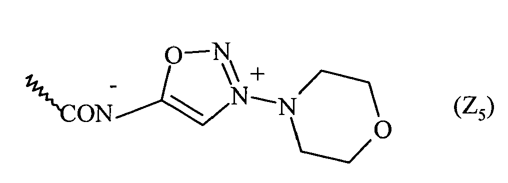 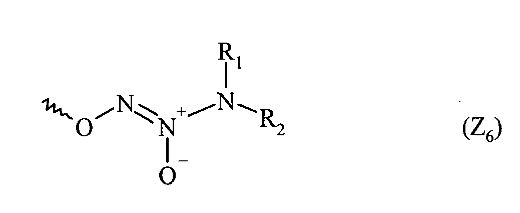 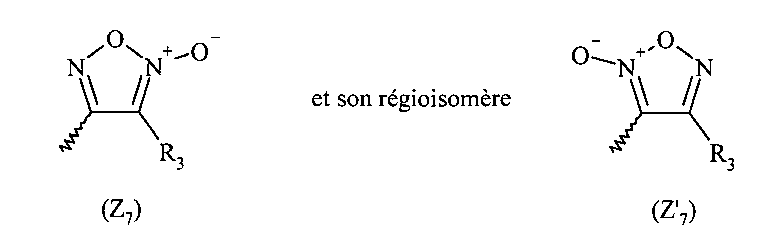 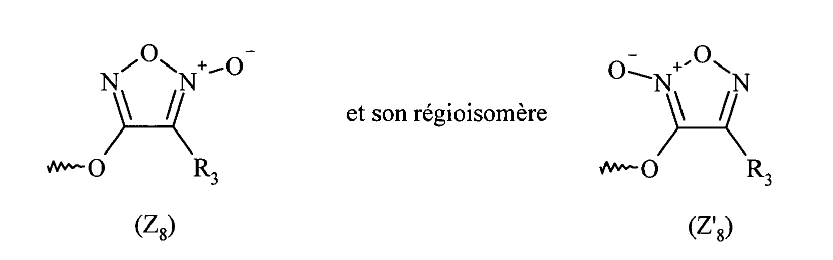 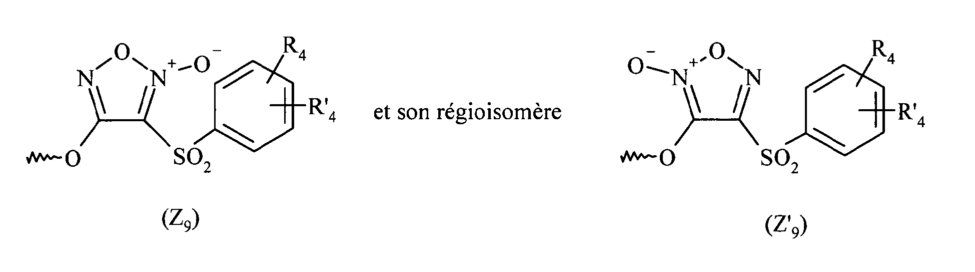 dans lesquels :
m représente 0 ou 1,
R₁ et R₂ , identiques ou différents, représentent chacun un groupement alkyle C₁-C₆ linéaire ou ramifié, ou bien R₁ et R₂ forment ensemble, avec l'atome d'azote qui les portent, un hétérocycle azoté comportant de 3 à 7 chaînons, éventuellement substitué par un groupement alkyle C₁-C₆ linéaire ou ramifié,
R₃ représente un groupement méthyle ou aminocarbonyle,
et R₄ et R'₄, identiques ou différents, représentent chacun un atome d'hydrogène ou d'halogène ou un groupement alkyle C₁-C₆ linéaire ou ramifié, trifluorométhyle ou trifluorométhoxy.

8. Composés de formule (I) selon l'une quelconque des revendications 1 à 4, dans laquelle B est choisi parmi les composés suivants :

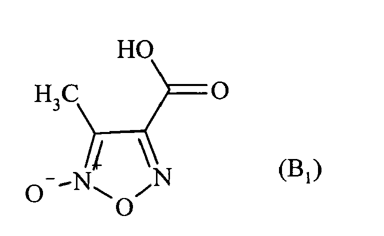

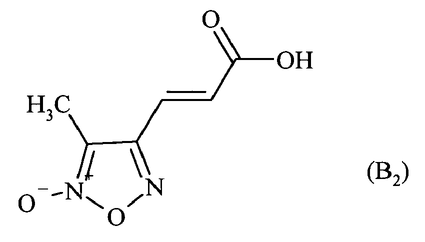

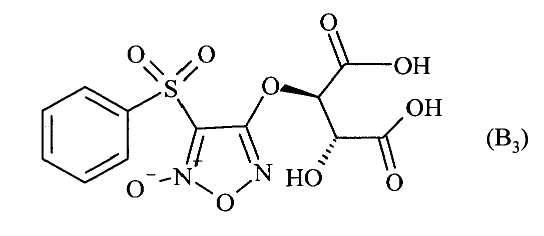

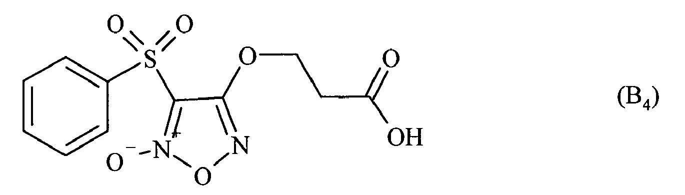

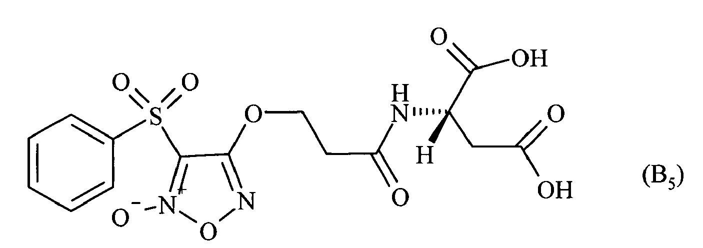

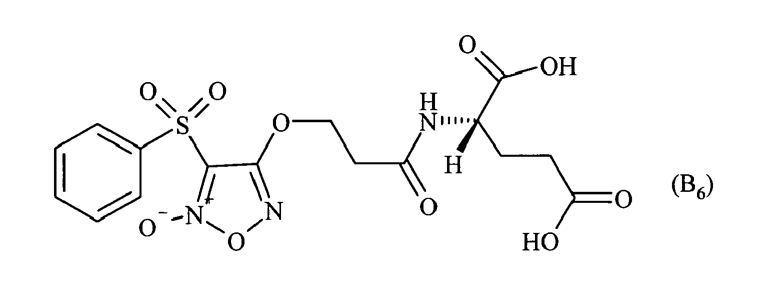

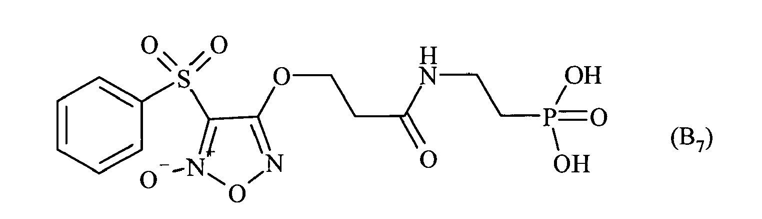

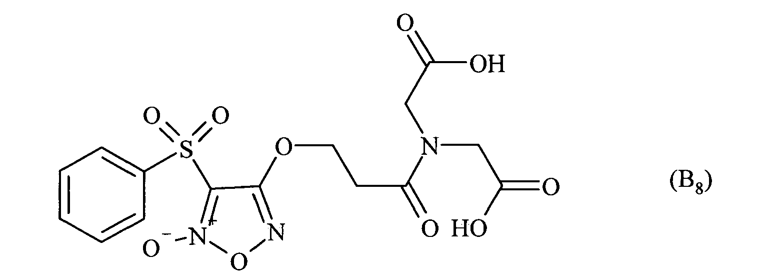

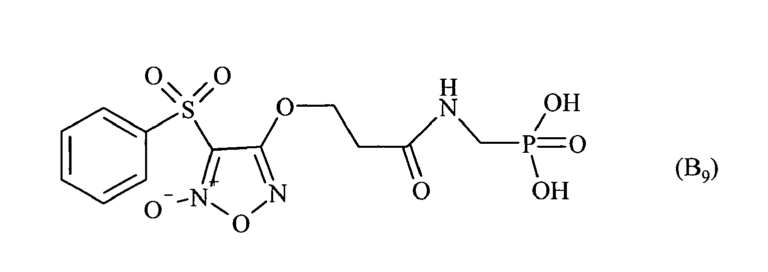

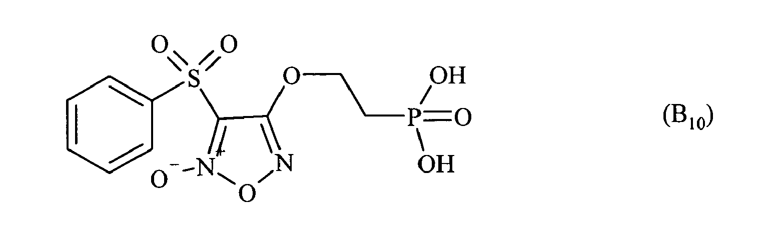

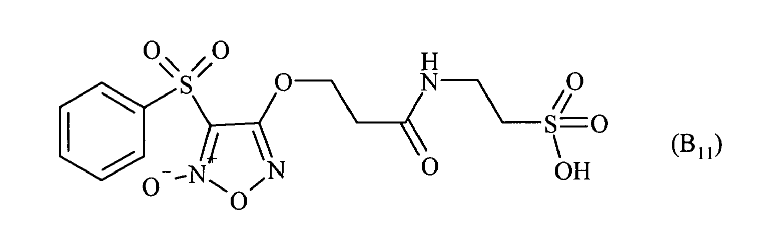

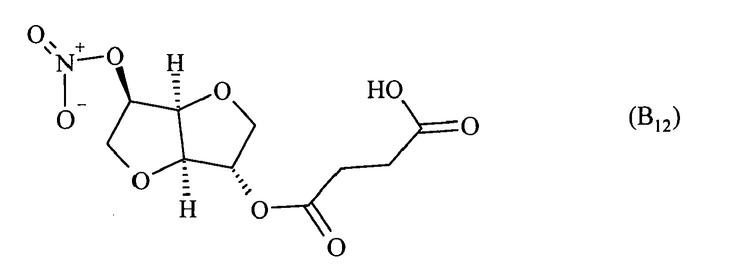

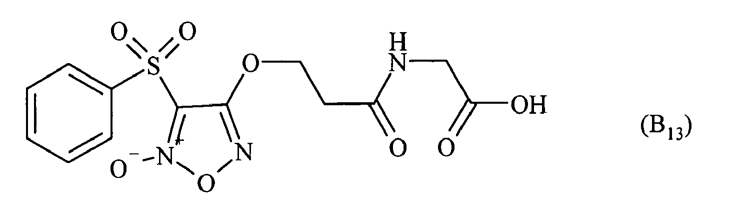

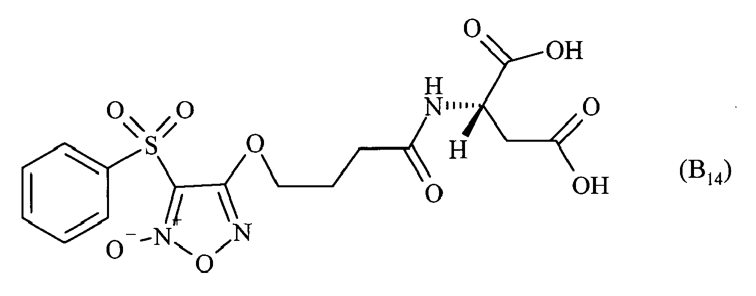

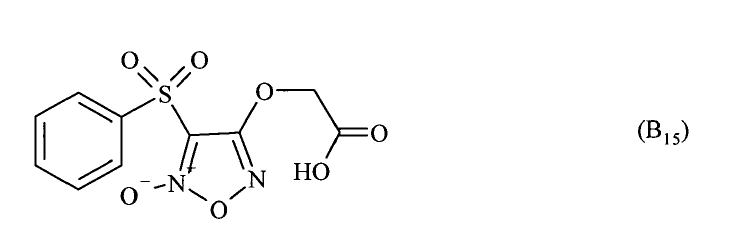

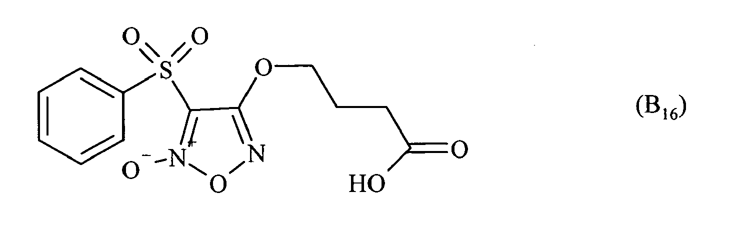

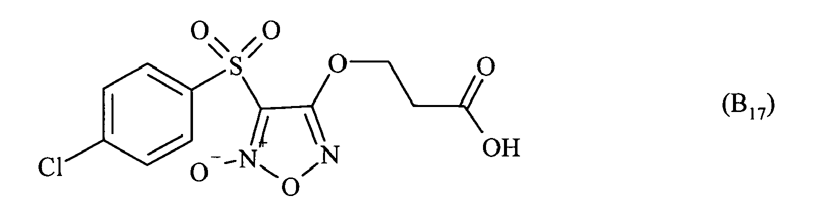

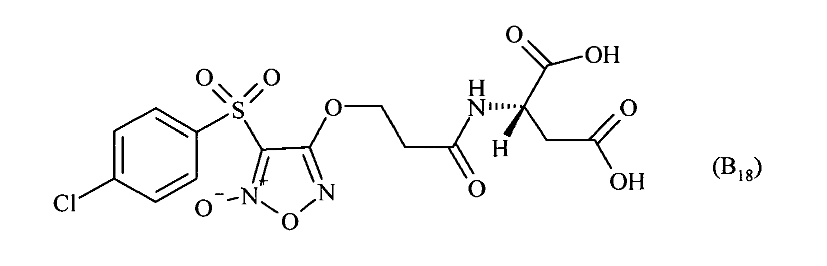

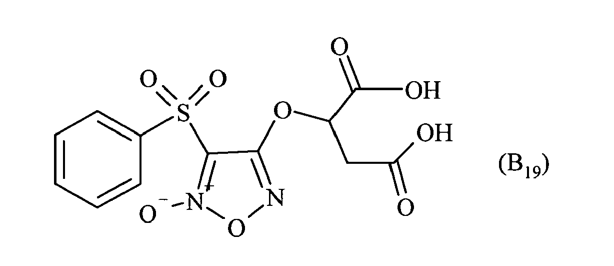

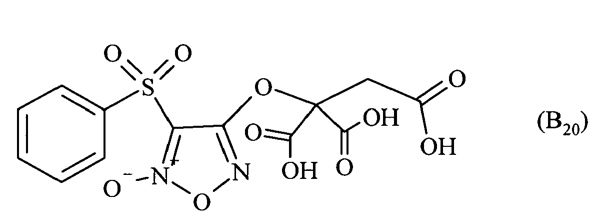

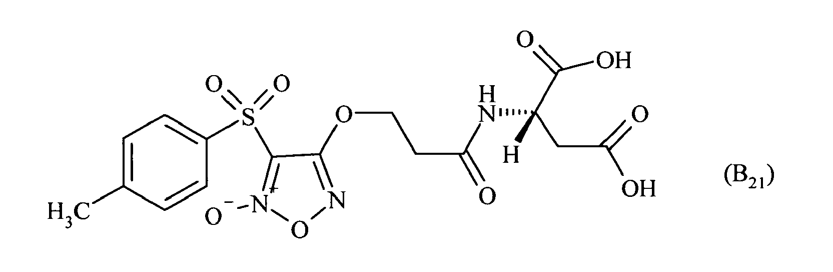

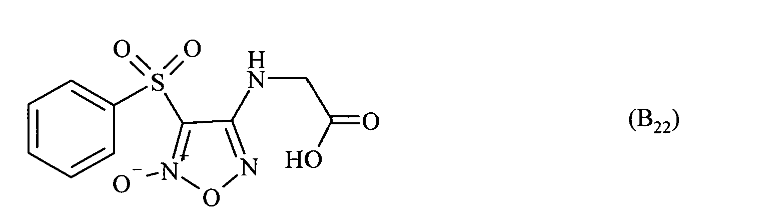

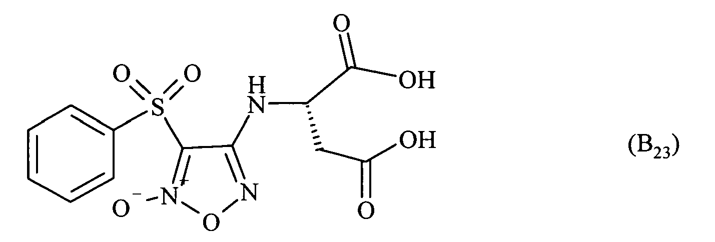

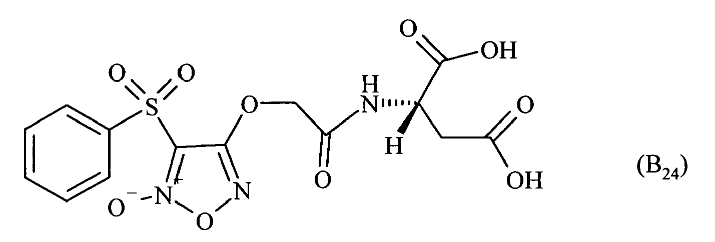

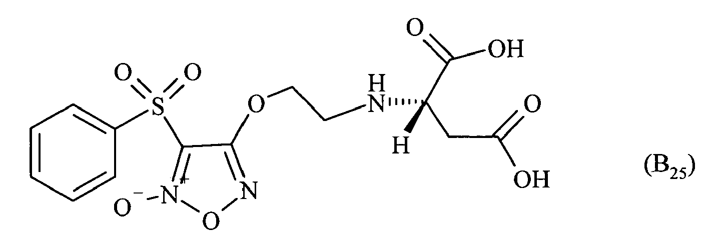

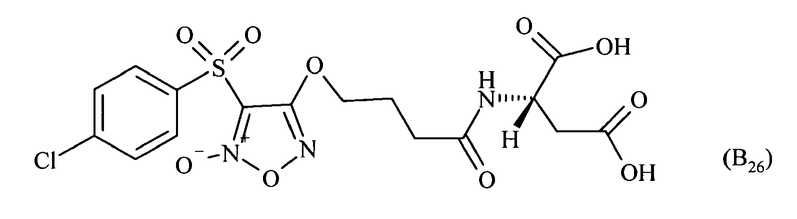

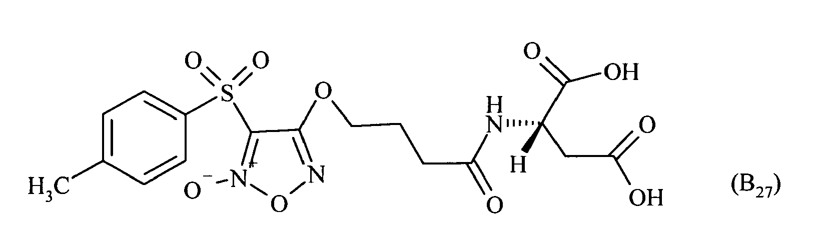

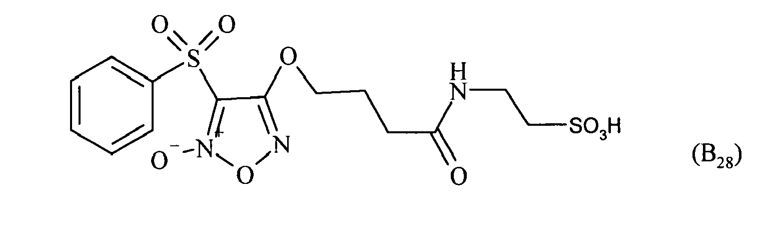

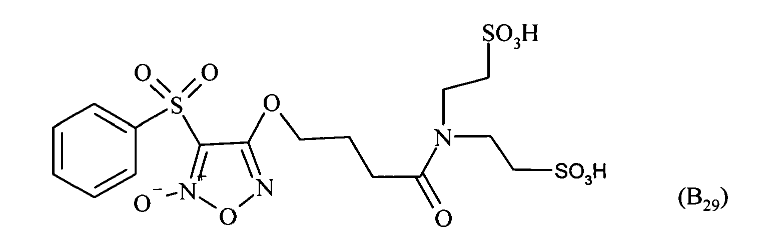

9. Procédé de synthèse des composés de formule (I) selon la revendication 1, dans lequel le composé A est mis en réaction avec le composé B en quantité au moins égale à (n/m) équivalents du composé A.

10. Composition pharmaceutique contenant comme principe actif un composé de formule (I) selon l'une quelconque des revendications 1 à 8, en combinaison avec un ou plusieurs excipients ou véhicules inertes, non toxiques, pharmaceutiquement acceptables.

11. Composition pharmaceutique selon la revendication 10 pour son utilisation dans le traitement des pathologies cardiovasculaires.

12. Composition pharmaceutique selon la revendication 11 pour son utilisation dans le traitement de l'hypertension artérielle et ses conséquences vasculaires et rénales, l'hypertension systolique, les maladies vasculaires périphériques, l'athérosclérose, la resténose, l'insuffisance cardiaque, la thrombose et tous les événements thromboemboliques, l'angine de poitrine stable ou instable, les accidents vasculaires cérébraux, les accidents coronariens, l'infarctus du myocarde, le remodelage vasculaire, le diabète et ses conséquences vasculaires et rénales, les complications liées aux interventions chirurgicales cardiovasculaires ou la dysfonction endothéliale.

13. Composition pharmaceutique selon la revendication 11 pour son utilisation dans le traitement des pathologies impliquant l'inflammation et le stress oxydant.
